# EUROPEAN PATENT SPECIFICATION

(11) **EP 1 471 919 B1**
(45) Date of publication and mention of the grant of the patent: **10.08.2005**
(21) Application number: 03706683.4
(22) Date of filing: 04.02.2003
(51) Int. Cl.: A61K 31/58, A61K 31/575, A61K 31/46, A61P 11/06, A61P 5/46

(54) **PHARMACEUTICAL COMPOSITIONS COMPRISING 17ALPHA-FURANYLESTERS OF 17BETA-CARBOTHIOATE ANDROSTANES WITH A MUSCARINIC RECEPTOR ANTAGONIST**
PHARMAZEUTISCHE ZUSAMMENSETZUNGEN ENTHALTEND 17ALPHA-FURANYLESTER VON 17BETA-CARBOTHIOAT ANDROSTANDERIVATEN UND EINEN MUSKARINREZEPTORANTAGONIST
COMPOSITIONS PHARMACEUTIQUES COMPRENANT 17ALPHA-FURANYLESTERS DE 17BETA-CARBOTHIOATE ANDROSTANES AVEC UN ANTAGONISTE DES RECEPTEURS MUSCARINIQUES

(30) Priority: 05.02.2002 GB 0202635
(43) Date of publication of application: 03.11.2004
(73) Proprietor: GLAXO GROUP LIMITED, Greenford, Middlesex UB6 0NN (GB)
(72) Inventor: BIGGADIKE, Keith GlaxoSmithKline, Stevenage, Hertfordshire SG1 2NY (GB)
(74) Representative: Pritchard, Judith
(86) International application number: PCT/GB2003/000491
(87) International publication number: WO 2003/066063

(56) References cited:
- EP-A- 0 418 716
- WO-A-01/04118
- WO-A-01/78736
- WO-A-01/78739
- WO-A-01/78741
- WO-A-02/00679
- WO-A-02/12265
- WO-A-97/05136
- WO-A-97/24365
- PETER J. BARNES: "Novel approaches and Targets for Treatment of Chronic Obstructive Pulmonary Disease" AMERICAN JOURNAL OF RESPIRATORY AND CRITICAL CARE MEDICINE, vol. 160, 1999, pages S72-S79, XP001098482 N.Y, USA
- PETER J. BARNES: "Efficacy of inhaled corticosteroids in Asthma" THE JOURNAL OF ALLERGY AND CLINICAL IMMUNOLOGY, vol. 102, no. 4, 1998, pages 531-538, XP000911075 usa
- B.J.O'CONNOR: "Combination Therapy" PULMONARY PHARMACOLOGY & THERAPEUTICS, vol. 11, no. 5/6, 1998, pages 397-399, XP000911059
- PETER J. BARNES: "Chronic Obstructive pulmonary disease: new opportunities for drug development" TRENDS IN PHARMACOLOGICAL SCIENCES,ELSEVIER TRENDS JOURNAL, vol. 19, no. 10, 1998, pages 415-423, XP004156947 Cambridge GB
- SIMON BOWLER: "Long acting beta agonists" AUSTRALIAN FAMILY PHYSICIAN, vol. 27, no. 12, 1998, pages 1114-1118, XP000973076

## Description

The present invention relates inter alia to pharmaceutical compositions containing a novel anti-inflammatory and an anti-allergic compound of the androstane series and to processes for their preparation. The present invention also relates to therapeutic uses thereof, particularly for the treatment of inflammatory and allergic conditions.

Glucocorticoids which have anti-inflammatory properties are known and are widely used for the treatment of inflammatory disorders or diseases such as asthma, chronic obstructive pulmonary disease (COPD) and rhinitis. For example, US Patent 4335121 discloses 6α, 9α-Difluoro-17α-(1-oxopropoxy)-11β-hydroxy-16α-methyl-3-oxo-androsta-1,4-diene-17β-carbothioic acid S-fluoromethyl ester (known by the generic name of fluticasone propionate) and derivatives thereof. The use of glucocorticoids generally, and especially in children, has been limited in some quarters by concerns over potential side effects. The side effects that are feared with glucocorticoids include suppression of the Hypothalamic-Pituitary-Adrenal (HPA) axis, effects on bone growth in children and on bone density in the elderly, ocular complications (cataract formation and glaucoma) and skin atrophy. Certain glucocorticoid compounds also have complex paths of metabolism wherein the production of active metabolites may make the pharmacodynamics and pharmacokinetics of such compounds difficult to understand. Whilst the modern steroids are very much safer than those originally introduced, it remains an object of research to produce new molecules which have excellent anti-inflammatory properties, with predictable pharmacokinetic and pharmacodynamic properties, with an attractive side effect profile, and with a convenient treatment regime.

Muscarinic receptor antagonists, especially those which are selective for M1 and/or M3 receptors over M2 receptors, have also found use in relief of inflammatory disorders of the lung, especially COPD and asthma. Muscarinic receptor antagonists may desirably be employed in therapy in combination with glucocorticoid compounds for the treatment of the aforementioned conditions.

We have now identified a novel composition comprising a novel glucocorticoid compound which substantially meets these objectives.

Thus, according to one aspect of the invention, there is provided a pharmaceutical composition comprising compound of formula (I) 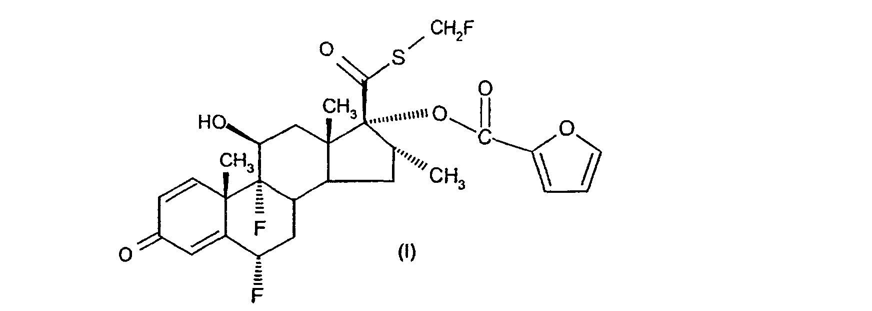 or a solvate thereof;
in combination with a muscarinic receptor antagonist (hereinafter a "composition of the invention").

The chemical name of the compound of formula (I) is 6α, 9α-Difluoro-17α-[(2-furanylcarbonyl)oxy]-11β-hydroxy-16α-methyl-3-oxo-androsta-1,4-diene-17β-carbothioic acid S-fluoromethyl ester.

References hereinafter to the compound according to the invention include both the compound of formula (I) and solvates thereof, particularly pharmaceutically acceptable solvates.

The compound of formula (I) and compositions containing it have potentially beneficial anti-inflammatory or anti-allergic effects, particularly upon topical administration, demonstrated by, for example, its ability to bind to the glucocorticoid receptor and to illicit a response via that receptor, with long acting effect. Hence, the compound of formula (I) is useful in the treatment of inflammatory and/or allergic disorders, especially in once-per-day therapy.

Preferably the muscarinic receptor antagonist is selective for the M1 and/or M3 receptors over the M2 receptor. Preferably the antagonist lacks significant M2 antagonist activity. In particular preferably the antagonist activity at M1 or M3 is at least 10 times that (more preferably 100 times that) of the antagonist activity at the

### M2 receptor. Preferably the antagonist is a more potent antagonist of M3 than M1. Preferably the antagonist has essentially no affinity for the M2 receptor.

Examples of muscarinic receptor antagonists include ipratropium salts, especially ipratropium bromide and oxitropium salts, especially oxitropium bromide. Preferably the muscarinic receptor antagonist is long acting, that it to say it has a duration of action sufficient to provide a therapeutic effect when dosed once or twice per day, especially once per day (i.e. it has a therapeutic effect over 24 hours). Example of a long acting muscarinic receptor antagonists include those described in EP 418716 (Boehringer lngelheim) such as tiotropium and salts thereof, especially tiotopium bromide. Other examples include those described in WO01/04118 (Almirall) such as those of formula (A) given below: 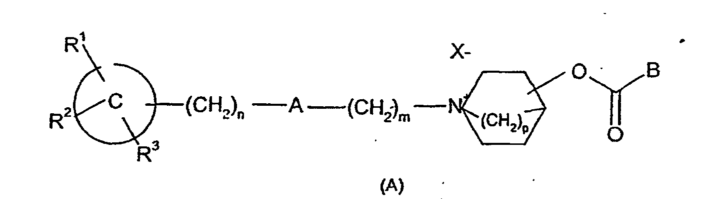 wherein:
C is a phenyl ring, a C₄ to C₉ heteroaromatic group containing one or more heteroatoms (preferably selected from nitrogen, oxygen and sulphur atoms), or a naphthalenyl, 5,6,7,8-tetrahydronaphthalenyl or biphenyl group;
R¹, R² and R³ each independently represent a hydrogen or halogen atom, or a hydroxy group, or a phenyl, -OR⁴, -SR⁴, -NR⁴R⁵, -NHCOR⁴, -CONR⁴R⁵,-CN, -NO₂, - COOR⁴ or -CF₃ group, or a straight or branched lower alkyl group which may optionally be substituted, for example, with a hydroxy or alkoxy group, wherein R⁴ and R⁵ each independently represent a hydrogen atom, straight or branched lower alkyl group, or together form an alicyclic ring; or R¹ and R² together form an aromatic, alicyclic or heterocyclic ring;
n is an integer from 0 to 4;

A represents a -CH₂-, -CH=CR⁶-, -CR⁶=CH-, -CR⁶R⁷-, -CO-, -O-, -S-, -S(O)-, SO₂or-NR⁶- group, wherein R⁶ and R⁷ each independently represent a hydrogen atom, straight or branched lower alkyl group, or R⁶ and R⁷ together form an alicyclic ring;
m is an integer from 0 to 8; provided that when m = 0, A is not -CH₂-; p is an integer from 1 to 2 and the substitution in the azoniabicyclic ring may be in the 2, 3 or 4 position including all possible configurations of the asymmetric carbons;
B represents a group of formula (i) or (ii): 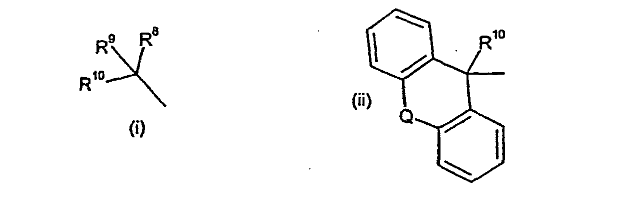 wherein R¹⁰ represents a hydrogen atom, a hydroxy or methyl group; and R⁸ and R⁹ each independently represents one of the following 5 moieties: 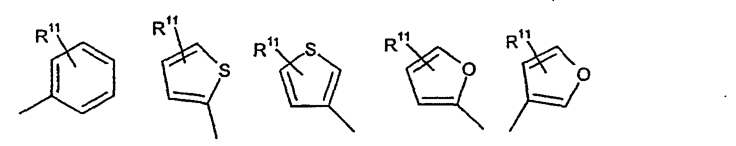 wherein R¹¹ represents a hydrogen or halogen atom, or a straight or branched lower alkyl group and Q represents a single bond, -CH₂-, -CH₂-CH₂-, -O-, -O-CH₂-, -S-, -S-CH₂- or -CH=CH-, and when (i) or (ii) contain a chiral centre they may represent either configuration;
X represent a pharmaceutically acceptable anion of a mono or polyvalent acid.

In the definition of compounds of formula (A), preferably lower alkyl means C₁₋₆alkyl, more preferably C₁₋₄alkyl. Preferably alicyclic and heterocyclic rings comprise 3-10, more preferably 5-7 members. Preferably aromatic rings contain 6-14 members, especially 6 or 10 members.

In the quaternary ammonium compounds of formula (A) an equivalent of anion (X-) is associated with the positive charge of the N atom. X may be an anion of various mineral acids. More preferably X is chloride, bromide or trifluoroacetate, especially bromide.

All stereoisomers at asymmetic centres are included within the definition of compounds of formula (A).

Preferred compounds of formula (A) are those wherein © represent a phenyl, pyrrolyl, or thienyl group; R¹, R² and R³ each independently represent a hydrogen atom, a hydroxy group or a halogen atom, wherein the halogen atom is preferably fluorine; n = 0 or 1; m is an integer from 1 to 6, particularly 1, 2 or 3; A represents a -CH₂-, -CH=CH- or -O- group.

In formula (A) it is also preferred that p = 2 and the substituent group -OC(O)B attached to the azoniabicyclo[2.2.2]octane is at the 3 position, preferably having the (R) configuration.

Especially preferred compounds are those wherein the -OC(O)B group in formula (A) is diphenylacetoxy, 2-hydroxy-2,2-diphenyl-acetoxy, 2,2-diphenylpropionyloxy, 2-hydroxy-2-phenyl-2-thien-2-yl-acetoxy, 2-furan-2-yl-2-hydroxy-2-phenylacetoxy, 2,2-dithien-2-ylacetoxy, 2-hydroxy-2,2-di-thien-2-ylacetoxy, 2-hydroxy-2,2-di-thien-3-ylacetoxy, 9-hydroxy-o[H]-fluorene-9-carbonyloxy,9-methyl-9[H]-fluorene-9-carbonyloxy, 9[H]-xanthene-9-carbonyloxy, 9-hydroxy-9[H]-xanthene-9-carbonyloxy or 9-methyl-9[H]-xanthene-9-carbonyloxy.

The most preferred compounds of formula (A) are those wherein the azoniabicyclo group is substituted on the nitrogen atom with a 3-phenoxypropyl, 2-phenoxyethyl, 3-phenylallyl, phenethyl, 4-phenylbutyl, 3-phenylpropyl, 3-[2-hydroxyphenoxy]propyl, 3-[4-fluorophenoxy]propyl, 2-benzyloxyethyl, 3-pyrrol-1-ylpropyl, 2-thien-2-ylethyl, 3-thien-2-ylpropyl, 3-phenylaminopropyl, 3-(methylphenylamino)propyl, 3-phenyls ulfanylpropyl,3-o-tolyloxypropyl,3-(2,4,6-trimethylphenoxy)propyl, 3-(2-tert-butyl-6-methylphenoxy)propyl, 3-(biphenyl-4-yloxy)propyl, 3-(5,6,7,8-tetrahydronaphthalen-2-yloxy)-propyl, 3-(naphthalen-2-yloxy)-propyl, 3-(naphthalen-1-yloxy)propyl, 3-(2-chlorophenoxy)propyl, 3-(2,4-difluorophenoxy)propyl, 3-(3-trifluoromethyl phenoxy)propyl, 3-(3-cyanophenoxy)propyl, 3-(4-cyanophenoxy)propyl, 3-(3-methoxyphenoxy)propyl, 3-(4-methoxyphenoxy)propyl, 3-(benzo[1,3]dioxol-5-yloxy)propyl, 3-(2-carbamoylphenoxy)propyl, 3-(3-dimethylaminophenoxy)propyl, 3-(4-nitrophenoxy)propyl, (3-nitrophenoxy)propyl, 3-(4-acetylaminophenoxy)propyl, 3-(3-methoxycarbonylphenoxy)propyl, 3-[4-(3-hydroxypropyl)phenoxy]propyl, 3-(2-hydroxymethylphenoxy)propyl, 3-(3-hydroxymethylphenoxy) propyl, 3-(4-hydroxyphenoxy)propyl, 3-(2-hydroxyphenoxy)propyl, 3-(4-hydroxyphenoxy)propyl, 3-(3-hydroxyphenoxy)propyl, 4-oxo-4-thien-2- ylbutyl, 3-(1-methyl-[1H]-imidazol-2-ylsulfanyl)propyl, 3-(benzothiazol-2-yloxy)propyl, 3-benzyloxypropyl, 6-(4-phenylbutoxy)hexyl, 4-phenoxybutyl, or 2-benzyloxypropyl. Especially preferred compounds are those wherein the azoniabicyclo group is substituted on the nitrogen atom with a 3-phenoxypropyl, 2-phenoxypropyl, 3-phenylallyl, phenethyl, 4-phenylbutyl, 3-phenylpropyl, 3-[2-hydroxyphenoxy]propyl, 3-[4-flurophenoxy]propyl, 2-benzyloxyethyl, 3-pyrrol-1-ylpropyl, 2-thien-2-ylethyl or 3-thien-2-ylpropyl group.

Compound (I) undergoes highly efficient hepatic metabolism to yield the 17-β carboxylic acid (X) as the sole major metabolite in rat and human *in vitro* systems. This metabolite has been synthesised and demonstrated to be >1000 fold less active than the parent compound in *in vitro* functional glucocorticoid assays.

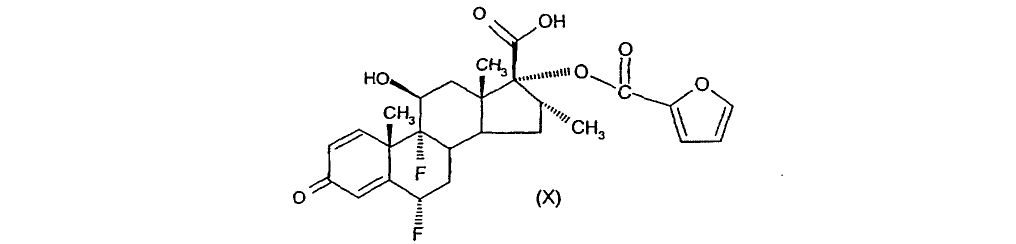

This efficient hepatic metabolism is reflected by *in vivo* data in the rat, which have demonstrated plasma clearance at a rate approaching hepatic blood flow and an oral bioavailability of <1%, consistent with extensive first-pass metabolism.

*In vitro* metabolism studies in human hepatocytes have demonstrated that compound (I) is metabolised in an identical manner to fluticasone propionate but that conversion of (I) to the inactive acid metabolite occurs approximately 5-fold more rapidly than with fluticasone propionate. This very efficient hepatic inactivation would be expected to minimise systemic exposure in man leading to an improved safety profile.

Inhaled steroids are also absorbed through the lung and this route of absorption makes a significant contribution to systemic exposure. Reduced lung absorption could therefore provide an improved safety profile. Studies with compound of formula (I) have shown significantly lower exposure to compound of formula (I) than with fluticasone propionate after dry powder delivery to the lungs of anaesthetised pigs.

An improved safety profile is believed to allow compositions containing the compound of formula (I) to demonstrate the desired anti-inflammatory effects when administered once-per day. Once-per-day dosing is considered to be significantly more convenient to patients than the twice-per day dosing regime that is normally employed for fluticasone propionate.

Examples of disease states in which the composition of the invention has utility include skin diseases such as eczema, psoriasis, allergic dermatitis, neurodermatitis, pruritis and hypersensitivity reactions; inflammatory conditions of the nose, throat or lungs such as asthma (including allergen-induced asthmatic reactions), rhinitis (including hayfever), nasal polyps, chronic obstructive pulmonary disease (COPD), interstitial lung disease, and fibrosis; inflammatory bowel conditions such as ulcerative colitis and Crohn's disease; and auto-immune diseases such as rheumatoid arthritis.

The composition of the invention may also have anti-tussive properties.

The composition of the invention is expected to be most useful in the treatment of inflammatory disorders of the respiratory tract eg asthma and COPD, particularly COPD.

It will be appreciated by those skilled in the art that reference herein to treatment extends to prophylaxis as well as the treatment of established conditions.

As mentioned above, the composition of the invention is useful in human or veterinary medicine, in particular as an anti-inflammatory and anti-allergic agent.

There is thus provided as a further aspect of the invention the composition of the invention for use in human or veterinary medicine, particularly in the treatment of patients with inflammatory and/or allergic conditions, especially for treatment once-per-day.

According to another aspect of the invention, there is provided the use of the composition of the invention for the manufacture of a medicament for the treatment of patients with inflammatory and/or allergic conditions, especially for treatment once-per-day.

In a further or alternative aspect, there is provided a method for the treatment of a human or animal subject with an inflammatory and/or allergic condition, which method comprises administering to said human or animal subject an effective amount of the composition of the invention especially for administration once-per-day.

The composition of the invention may be formulated for administration in any convenient way, and the invention therefore also includes within its scope pharmaceutical compositions comprising the compound of formula (I) or a physiologically acceptable solvate thereof, a muscarinic receptor antagonist together, if desirable, in admixture with one or more physiologically acceptable diluents or carriers. Pharmaceutical compositions suitable for once-per-day administration are of particular interest. Formulations are preferably administered by inhalation topically to the lung.

Further, there is provided a process for the preparation of such pharmaceutical compositions which comprises mixing the ingredients.

The compound according to the invention may, for example, be formulated for oral, buccal, sublingual, parenteral, local or rectal administration, especially local administration.

Local administration as used herein, includes administration by insufflation and inhalation. Examples of various types of preparation for local administration include ointments, lotions, creams, gels, foams, preparations for delivery by transdermal patches, powders, sprays, aerosols, capsules or cartridges for use in an inhaler or insufflator or drops (eg eye or nose drops), solutions/suspensions for nebulisation, suppositories, pessaries, retention enemas and chewable or suckable tablets or pellets (eg for the treatment of aphthous ulcers) or liposome or microencapsulation preparations.

Advantageously compositions for topical administration to the lung include dry powder compositions and spray compositions.

Dry powder compositions for topical delivery to the lung by inhalation may, for example, be presented in capsules and cartridges for use in an inhaler or insufflator of, for example, gelatine. Formulations generally contain a powder mix for inhalation of the compound of the invention and a suitable powder base (carrier substance) such as lactose or starch. Use of lactose is preferred. Each capsule or cartridge may generally contain between 20µg-10mg of the compound of formula (I) and the muscarinic receptor antagonist. Alternatively, the compound of the invention may be presented without excipients. Packaging of the formulation may be suitable for unit dose or multi-dose delivery. In the case of multi-dose delivery, the formulation can be pre-metered (eg as in Diskus, see GB 2242134 or Diskhaler, see GB 2178965, 2129691 and 2169265) or metered in use (eg as in Turbuhaler, see EP 69715). An example of a unit-dose device is Rotahaler (see GB 2064336). The Diskus inhalation device comprises an elongate strip formed from a base sheet having a plurality of recesses spaced along its length and a lid sheet hermetically but peelably sealed thereto to define a plurality of containers, each container having therein an inhalable formulation containing a compound of formula (I) and the muscarinic receptor antagonist preferably combined with lactose. Preferably, the strip is sufficiently flexible to be wound into a roll. The lid sheet and base sheet will preferably have leading end portions which are not sealed to one another and at least one of the said leading end portions is constructed to be attached to a winding means. Also, preferably the hermetic seal between the base and lid sheets extends over their whole width. The lid sheet may preferably be peeled from the base sheet in a longitudinal direction from a first end of the said base sheet.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the compound of formula (I) and the muscarinic receptor antagonist and a suitable propellant such as a fluorocarbon or hydrogen-containing chlorofluorocarbon or mixtures thereof, particularly hydrofluoroalkanes, especially 1,1,1,2-tetrafluoroethane, 1,1,1,2,3,3,3-heptafluoro-n-propane or a mixture thereof. The aerosol composition may optionally contain additional formulation excipients well known in the art such as surfactants eg oleic acid or lecithin and cosolvents eg ethanol. One example formulation is excipient free and consists essentially of (eg consists of) compound of formula (I) (preferably in unsolvated form eg as Form 1), a muscarinic receptor antagonist (optionally together with a further active ingredient) and a propellant selected from 1,1,1,2-tetrafluoroethane, 1,1,1,2,3,3,3-heptafluoro-n-propane and mixture thereof. Another example formulation comprises particulate compound of formula (I), a propellant selected from 1,1,1,2-tetrafluoroethane, 1,1,1,2,3,3,3-heptafluoro-n-propane and mixture thereof and a suspending agent which is soluble in the propellant eg an oligolactic acid or derivative thereof as described in WO94/21229. The preferred propellant is 1,1,1,2-tetrafluoroethane. Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10µm, preferably 2-5µm. Particles having a size above 20µm are generally too large when inhaled to reach the small airways. To achieve these particle sizes the particles of compound of formula (I) and the muscarinic receptor antagonist (and any further therapeutically active ingredient) as produced may be size reduced by conventional means eg by micronisation. The desired fraction may be separated out by air classification or sieving. Preferably, the particles will be crystalline, prepared for example by a process which comprises mixing in a continuous flow cell in the presence of ultrasonic radiation a flowing solution of compound of formula (I) and the muscarinic receptor antagonist as medicament (either separately or together) in a liquid solvent with a flowing liquid antisolvent for said medicament (eg as described in International Patent Application PCT/GB99/04368) or else by a process which comprises admitting a stream of solution of the substance in a liquid solvent and a stream of liquid antisolvent for said substance tangentially into a cylindrical mixing chamber having an axial outlet port such that said streams are thereby intimately mixed through formation of a vortex and precipitation of crystalline particles of the substance is thereby caused (eg as described in International Patent Application PCT/GB00/04327). When an excipient such as lactose is employed, generally, the particle size of the excipient will be much greater than the inhaled medicament within the present invention. When the excipient is lactose it will typically be present as milled lactose, wherein not more than 85% of lactose particles will have a MMD of 60-90µm and not less than 15% will have a MMD of less than 15µm.

Formulations for administration topically to the lung or nose include pressurised aerosol formulations and aqueous formulations administered to the nose by pressurised pump. Aqueous formulations for administration to the lung or nose may be provided with conventional excipients such as buffering agents, tonicity modifying agents and the like. Aqueous formulations may also be administered to the lung and nose by nebulisation.

Other possible presentations include the following:

Ointments, creams and gels, may, for example, be formulated with an aqueous or oily base with the addition of suitable thickening and/or gelling agent and/or solvents. Such bases may thus, for example, include water and/or an oil such as liquid paraffin or a vegetable oil such as arachis oil or castor oil, or a solvent such as polyethylene glycol. Thickening agents and gelling agents which may be used according to the nature of the base include soft paraffin, aluminium stearate, cetostearyl alcohol, polyethylene glycols, woolfat, beeswax, carboxypolymethylene and cellulose derivatives, and/or glyceryl monostearate and/or non-ionic emulsifying agents.

Lotions may be formulated with an aqueous or oily base and will in general also contain one or more emulsifying agents, stabilising agents, dispersing agents, suspending agents or thickening agents.

Powders for external application may be formed with the aid of any suitable powder base (carrier substance), for example, talc, lactose or starch. Drops may be formulated with an aqueous or non-aqueous base also comprising one or more dispersing agents, solubilising agents, suspending agents or preservatives.

If appropriate, the formulations of the invention may be buffered by the addition of suitable buffering agents.

The proportion of the active compound of formula (I) and the muscarinic receptor antagonist in the local compositions according to the invention depends on the precise type of formulation to be prepared but will generally be within the range of from 0.001 to 10% by weight. Generally, however for most types of preparations advantageously the proportion used will be within the range of from 0.005 to 1%. However, in powders for inhalation or insufflation the proportion used will usually be within the range of from 0.01 to 5%.

Aerosol formulations are preferably arranged so that each metered dose or "puff" of aerosol contains 1µg-2000µg eg 20µg-2000µg, preferably about 20µg-500µg of a compound of formula (I). Administration may be once daily or several times daily, for example 2, 3, 4 or 8 times, giving for example 1, 2 or 3 doses each time. Preferably the compound of formula (I) is delivered once or twice daily, more preferably once-per-day. The overall daily dose with an aerosol will typically be within the range 10µg-10mg eg 100µg-10mg preferably, 200µg-2000µg.

Muscarinic receptor antagonists will be dosed in an amount depending on the potency and safety of the compound involved. For example ipratropium and salts thereof (eg ipratropium bromide) is usually dosed at around 40µg -320µg per day (say 1-8 puffs of 40µg per day); oxitropium and salts thereof (eg oxitropium bromide) is usually dosed at around 100µg -640µg per day (say 1-6 puffs of 100µg per day); tiotropium and salts thereof (eg tiotropium bromide) is usually dosed at around 10µg - 50µg per day (say 1-4 puffs of 10µg per day); compounds of formula (A) may be dosed in an amount of 100µg -1200µg per day.

Since the compound of formula (I) is long-acting, preferably the composition of the invention will be delivered once-per-day and the dose of each of the active ingredients will be selected so that the compound has a therapeutic effect in the treatment of respiratory disorders (eg asthma or COPD, particularly asthma) over 24 hours or more.

Topical preparations may be administered by one or more applications per day to the affected area; over skin areas occlusive dressings may advantageously be used. Continuous or prolonged delivery may be achieved by an adhesive reservoir system.

For internal administration the compound according to the invention may, for example, be formulated in conventional manner for oral, parenteral or rectal administration. Formulations for oral administration include syrups, elixirs, powders, granules, tablets and capsules which typically contain conventional excipients such as binding agents, fillers, lubricants, disintegrants, wetting agents, suspending agents, emulsifying agents, preservatives, buffer salts, flavouring, colouring and/or sweetening agents as appropriate. Dosage unit forms are, however, preferred as described below.

Preferred forms of preparation for internal administration are dosage unit forms i.e. tablets and capsules. Such dosage unit forms contain from 0.1mg to 20mg preferably from 2.5 to 10mg of the composition of the invention.

The compound according to the invention may in general may be given by internal administration in cases where systemic adreno-cortical therapy is indicated.

In general terms preparations, for internal administration may contain from 0.05 to 10% of the active ingredient dependent upon the type of preparation involved. The daily dose may vary from 0.1mg to 60mg, eg 5-30mg, dependent on the condition being treated, and the duration of treatment desired.

Slow release or enteric coated formulations may be advantageous, particularly for the treatment of inflammatory bowel disorders.

The pharmaceutical compositions according to the invention may also be used in combination with another therapeutically active agent, for example, a β₂ adrenoreceptor agonist, an anti-histamine or an anti-allergic. The invention thus provides, in a further aspect, a combination comprising the composition of the invention together with another therapeutically active agent, for example, a β₂-adrenoreceptor agonist, an anti-histamine or an anti-allergic.

Examples of β₂-adrenoreceptor agonists include salmeterol (eg as racemate or a single enantiomer such as the R-enantiomer), salbutamol, formoterol, salmefamol, fenoterol or terbutaline and salts thereof, for example the xinafoate salt of salmetecol, the sulphate salt or free base of salbutamol or the fumarate salt of formoterol.

Pharmaceutical compositions employing compositions of the invention in combination with long-acting β₂-adrenoreceptor agonists are particularly preferred, especially those which have a therapeutic effect (eg in the treatment of asthma or COPD, particularly asthma) over 24 hours or more.

Particularly preferred long acting β₂-adrenoreceptor agonists include those described in WO 02066422, WO02070490 and WO02076933.

Especially preferred long-acting β₂-adrenoreceptor agonists include compounds of formula(M): 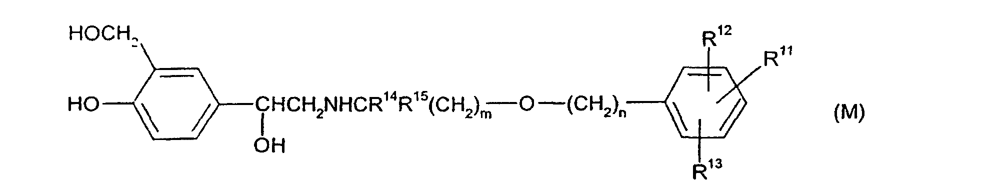 or a salt or solvate thereof, wherein:
m is an integer of from 2 to 8;
n is an integer of from 3 to 11,
with the proviso that m + n is 5 to 19,
R¹¹ is -XSO₂NR¹⁶R¹⁷ wherein X is -(CH₂)ₚ- or C₂₋₆ alkenylene;
R¹⁶ and R¹⁷ are independently selected from hydrogen, C₁₋₆alkyl, C₃₋₇cycloalkyl, C(O)NR¹⁸R¹⁹, phenyl, and phenyl (C₁₋₄alkyl)-,
or R¹⁶ and R¹⁷, together with the nitrogen to which they are bonded, form a 5-, 6-, or 7- membered nitrogen containing ring, and R¹⁶ and R¹⁷ are each optionally substituted by one or two groups selected from halo, C₁₋₆alkyl, C₁₋₆haloalkyl, C₁₋₆alkoxy, hydroxy-substituted C₁₋₆alkoxy, -CO₂R¹⁸, -SO₂NR¹⁸R¹⁹, -CONR¹⁸R¹⁹, -NR¹⁸C(O)R¹⁹, or a 5-, 6- or 7-membered hcterocylic ring;
R¹⁸ and R¹⁰ are independently selected from hydrogen, C₁₋₆alkyl, C₃₋₆cycloalkyl, phenyl, and phenyl (C₁₋₄alkyl)-; and
p is an integer of from 0 to 6, preferably from 0 to 4;
R¹² and R¹³ are independently selected from hydrogen, C₁₋₆alkyl, C₁₋₆alkoxy, halo, phenyl, and C₁₋₆haloalkyl; and
R¹⁴ and R¹⁵ are independently selected from hydrogen and C₁₋₄alkyl with the proviso that the total number of carbon atoms in R¹⁴ and R¹⁵ is not more than 4.

Since the compound of formula (I) is long-acting, preferably the composition comprising the compound of formula (I), the muscarinic receptor antagonist (especially a long acting muscarinic receptor antagonist) and the long-acting β₂-adrenoreceptor agonists will be delivered once-per-day and the dose of each will be selected so that the composition has a therapeutic effect in the treatment of respiratory disorders effect (eg in the treatment of asthma or COPD, particularly asthma) over 24 hours or more.

Examples of anti-histamines include methapyrilene or loratadine.

Other suitable combinations include, for example, other anti-inflammatory agents eg. NSAIDs (eg. PDE4 inhibitors, leukotriene antagonists, iNOS inhibitors, tryptase and elastase inhibitors, beta-2 integrin antagonists and adenosine 2a agonists)) or antiinfective agents (eg. antibiotics, antivirals).

Of particular interest is use of the compounds of formula (I) in combination with a phosphodiesterase 4 (PDE4) inhibitor. The PDE4-specific inhibitor useful in this aspect of the invention may be any compound that is known to inhibit the PDE4 enzyme or which is discovered to act as a PDE4 inhibitor, and which are only PDE4 inhibitors, not compounds which inhibit other members of the PDE family as well as PDE4. Generally it is preferred to use a PDE4 inhibitor which has an IC₅₀ ratio of about 0.1 or greater as regards the IC₅₀ for the PDE4 catalytic form which binds rolipram with a high affinity divided by the IC₅₀ for the form which binds rolipram with a low affinity. For the purposes of this disclosure, the cAMP catalytic site which binds R and S rolipram with a low affinity is denominated the "low affinity" binding site (LPDE 4) and the other form of this catalytic site which binds rolipram with a high affinity is denominated the "high affinity" binding site (HPDE 4). This term "HPDE4" should not be confused with the term "hPDE4" which is used to denote human PDE4.

A method for determining IC50 ratios is set out in US patent 5 998 428 which is herein incorporated in full by reference as though set out herein. See also PCT application WO 00/51599 for another description of the assay.

The preferred PDE4 inhibitors of use in this invention will be those compounds which have a salutary therapeutic ratio, i.e., compounds which preferentially inhibit cAMP catalytic activity where the enzyme is in the form that binds rolipram with a low affinity, thereby reducing the side effects which apparently are linked to inhibiting the form which binds rolipram with a high affinity. Another way to state this is that the preferred compounds will have an IC₅₀ ratio of about 0.1 or greater as regards the IC₅₀ for the PDE4 catalytic form which binds rolipram with a high affinity divided by the IC₅₀ for the form which binds rolipram with a low affinity.

A further refinement of this standard is that of one wherein the PDE4 inhibitor has an IC₅₀ ratio of about 0.1 or greater; said ratio is the ratio of the IC₅₀ value for competing with the binding of 1nM of [³H]R-rolipram to a form of PDE4 which binds rolipram with a high affinity over the IC₅₀ value for inhibiting the PDE4 catalytic activity of a form which binds rolipram with a low affinity using 1 µM[³H]-cAMP as the substrate.

Most preferred are those PDE4 inhibitors which have an IC₅₀ ratio of greater than 0.5, and particularly those compounds having a ratio of greater than 1.0. Preferred compounds are cis 4-cyano-4-(3-cyclopentyloxy-4-methoxyphenyl)cyclohexan-1-carboxylic acid, 2-carbomethoxy-4-cyano-4-(3-cyclopropylmethoxy-4-difluoromethoxyphenyl)cyclohexan-1-one and *cis*-[4-cyano-4-(3-cyclopropylmethoxy-4-difluoromethoxyphenyl)cyclohexan-1-ol]; these are examples of compounds which bind preferentially to the low affinity binding site and which have an IC₅₀ ratio of 0.1 or greater.

Other compounds of interest include:

Compounds set out in U.S. patent 5,552,438 issued 03 September, 1996; this patent and the compounds it discloses are incorporated herein in full by reference. The compound of particular interest, which is disclosed in U.S. patent 5,552,438, iscis-4-cyano-4-[3- (cyclopentyloxy)-4-methoxyphenyl]cyclohexane-1-carboxylic acid (also known as cilomalast) and its salts, esters, pro-drugs or physical forms; AWD-12-281 from elbion (Hofgen, N. et al. 15th EFMC Int Symp Med Chem (Sept 6-10, Edinburgh) 1998, Abst P.98); a 9-benzyladenine derivative nominated NCS-613 (INSERM); D-4418 from Chiroscience and Schering-Plough; a benzodiazepine PDE4 inhibitor identified as CI-1018 (PD-168787; Parke-Davis/Warner-Lambert); a benzodioxote derivative Kyowa Hakko disclosed in WO 9916766; V-11294A from Napp (Landells, L.J. et al. Eur Resp J [Annu Cong Eur Resp Soc (Sept 19-23, Geneva) 1998] 1998, 12(Suppl. 28): Abst P2393); roflumilast (CAS reference No 162401-32-3) and a pthalazinone (WO 9947505) from Byk-Gulden; or a compound identified as T-440 (Tanabe Seiyaku; Fuji, K. et al. J Pharmacol Exp Ther,1998, 284(1): 162).

The invention thus provides, in a further aspect, a combination comprising the composition of the invention together with a PDE4 inhibitor.

The combination referred to above may conveniently be presented for use in the form of a pharmaceutical formulation and thus pharmaceutical formulations comprising a combination as defined above together with a physiologically acceptable diluent or carrier represent a further aspect of the invention.

The compound according to the invention in combination with another therapeutically active ingredient as described above may be formulated for administration in any convenient way, and the invention therefore also includes within its scope pharmaceutical compositions comprising the composition of the invention in combination with another therapeutically active ingredient together, if desirable, in admixture with one or more physiologically acceptable diluents or carriers. The preferred route of administration for inflammatory disorders of the respiratory tract will generally be administration by inhalation.

The pharmaceutical composition of the invention may further comprise one or more excipients.

By the term "excipient", as used herein, it is meant to mean substantially inert materials that are nontoxic and do not interact with other components of a composition in a deleterious manner including, but not limited to, pharmaceutical grades of: carbohydrates, organic and inorganic salts, polymers, amino acids, phospholipids, wetting agents, emulsifiers, surfactants, poloxamers, pluronics, and ion exchange resins, and combinations thereof, a non-exhaustive list of examples of which are provided below:
Carbohydrates, including: monosaccharides, such as, but not limited to, fructose ; disaccharides, such as, but not limited to lactose, and combinations and derivatives thereof; polysaccharides, such as, but not limited to, cellulose and combinations and derivatives thereof; oligosaccharides, such as, but not limited to, dextrins, and combinations and derivatives thereof; polyols, such as but not limited to sorbitol, and combinations and derivatives thereof;
Organic and inorganic salts, including but not limited to sodium or calcium phosphates, magnesium stearate, and combinations and derivatives thereof;
Polymers, including: natural biodegradable protein polymers including, but not limited to, gelatin and combinations and derivatives thereof; Natural biodegradable polysaccharide polymers including, but not limited to, chitin and starch, crosslinked starch, and combinations and derivatives thereof; Semisynthetic biodegradable polymers including, but not limited to, derivatives of chitosan; Synthetic biodegradable polymers including but not limited to polyethylene glycols (PEG), polylactic acid (PLA), synthetic polymers including but not limited to polyvinyl alcohol and combinations and derivatives thereof;
Amino acids including but not limited to including non-polar amino acids, such as leucine and combinations and derivatives thereof;
Phospholipids, including lecithins and combinations and derivatives thereof;
Wetting agents/ Surfactants/Emulsifiers, including, but not limited to gum acacia, cholesterol, fatty acids including, combinations and derivatives thereof;
Poloxamers/ Pluronics: including but not limited to poloxamer 188, Pluronic® F-108, and combinations and derivations thereof;
Ion exchange resins: including but not limited to amberlite IR120 and combinations and derivatives thereof;
and combinations of the noted excipients.

Further, there is provided a process for the preparation of such pharmaceutical compositions which comprises mixing the ingredients.

Appropriate doses of known therapeutic agents will be readily appreciated by those skilled in the art.

Compounds may be tested for their muscarinic receptor antagonist activity by reference to methods disclosed in E-B Haddad et al (1994) J Mol Pharmacol 45, 899 and references cited therein.

Compounds of formula (A) may be prepared following methods described in WO01/04118. Tiotropium, ipratropium and oxitropium and salts thereof may be prepared following methods described in Merck Index.

A process for preparing a compound of formula (I) comprises alkylation of a thioacid of formula (II) 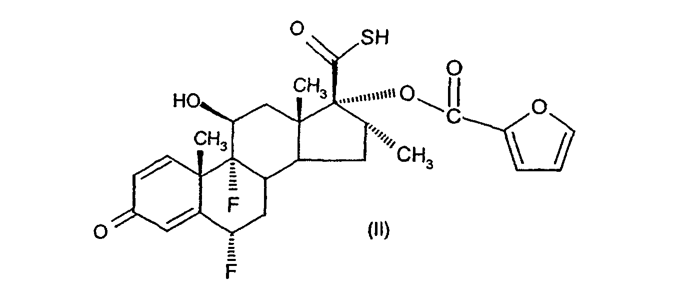 or a salt thereof.

In this process the compound of formula (II) may be reacted with a compound of formula FCH₂L wherein L represents a leaving group (eg a halogen atom, a mesyl or tosyl group or the like) for example, an appropriate fluoromethyl halide under standard conditions. Preferably, the fluoromethyl halide reagent is bromofluoromethane. Preferably the compound of formula (II) is employed as a salt, particularly the salt with diisopropylethylamine.

In a preferred process for preparing the compound of formula (I), the compound of formula (II) or a salt thereof is treated with bromofluoromethane optionally in the presence of a phase transfer catalyst. A preferred solvent is methylacetate, or more preferably ethylacetate, optionally in the presence of water. The presence of water improves solubility of both starting material and product and the use of a phase transfer catalyst results in an increased rate of reaction. Examples of phase transfer catalysts that may be employed include (but are not restricted to) tetrabutylammonium bromide, tetrabutylammonium chloride, benzyltributylammonium bromide, benzyltributylammonium chloride, benzyltriethylammonium bromide, methyltributylammonium chloride and methyltrioctylammonium chloride. THF has also successfully been employed as solvent for the reaction wherein the presence of a phase transfer catalyst again provides a significantly faster reaction rate. Preferably the product present in an organic phase is washed firstly with aqueous acid eg dilute HCI in order to remove amine compounds such as triethylamine and diisopropylethylamine and then with aqueous base eg sodium bicarbonate in order to remove any unreacted precursor compound of formula (II).

Compound of formula (I) in unsolvated form may be prepared by a process comprising:
(a) Crystallising the compound of formula (I) in the presence of a non-solvating solvent such as ethanol, methanol, water, ethyl acetate, toluene, methylisobutylketone or mixtures thereof; or
(b) Desolvafing a compound of formula (I) in solvated form (eg in the form of a solvate with acetone, isopropanol, methylethylketone, DMF or tetrahydrofuran) eg by heating.

In step (b) the desolvation will generally be performed at a temperature exceeding 50 °C preferably at a temperature exceeding 100°C. Generally heating will be performed under vacuum.

Compound of formula (I) in unsolvated form has been found to exist in 3 crystalline polymorphic forms, Forms 1, 2 and 3, although Form 3 may be an unstable variant of Form 2. The Forms are characterised by their X-ray diffraction (XRPD) patterns Broadly speaking the Forms are characterised in their XRPD profiles as follows:
Form 1: Peak at around 18.9 degrees 2Theta
Form 2: Peaks at around 18.4 amd 21.5 degrees 2Theta
Form 3: Peaks at around 18.6 and 19.2 degrees 2Theta.

Forms 1 appears likely to be the thermodynamically most stable form since Forms 2 and 3 are converted into Form 1 on heating.

Preferably compositions of the invention employ the compound of formula (I) in unsolvated form, especially as polymorph Form 1.

A process for preparing a compound of formula (I) as unsolvated Form 1 polymorph comprises dissolving compound of formula (I) in methylisobutylketone, ethyl acetate or methyl acetate and producing compound of formula (I) as unsolvated Form 1 by addition of a non-solvating anti-solvent such as iso-octane or toluene.

According to a first preferred embodiment of this process the compound of formula (I) may be dissolved in ethyl acetate and compound of formula (I) as unsolvated Form 1 polymorph may be obtained by addition of toluene as anti-solvent. In order to improve the yield, preferably the ethyl acetate solution is hot and once the toluene has been added the mixture is distilled to reduce the content of ethyl acetate.

According to a second preferred embodiment of this process the compound of formula (I) may be dissolved in methylisobutylketone and compound of formula (I) as unsolvated Form 1 polymorph may be obtained by addition of isooctane as anti-solvent

Compound of formula (I) in solvated form may be prepared by crystallising the compound of formula (I) from a solvating solvent such as acetone or tetrahydrofuran (THF).

Compounds of formula (II) may be prepared from the corresponding 17α-hydroxyl derivative of formula (III): 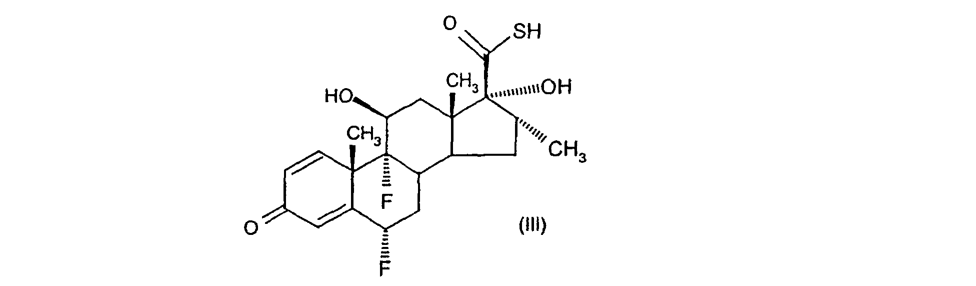 using for example, the methodology described by G. H. Phillipps et al., (1994) Journal of Medicinal Chemistry, 37, 3717-3729. For example the step typically comprises the addition of a reagent suitable for performing the esterification eg an activated derivative of 2-furoic acid such as an activated ester or preferably a 2-furoyl halide eg 2-furoyl chloride (employed in at least 2 times molar quantity relative to the compound of formula (III)) in the presence of an organic base eg triethylamine. The second mole of 2-furoyl chloride reacts with the thioacid moiety in the compound of formula (III) and needs to be removed eg by reaction with an amine such as diethylamine.

This method suffers disadvantages, however, in that the resultant compound of formula (II) is not readily purified of contamination with the by-product 2-furoyldiethylamide. We have therefore invented several improved processes for performing this conversion.

In a first such improved process we have discovered that by using a more polar amine such as diethanolamine, a more water soluble by-product is obtained (in this case 2-furoyldiethanolamide) which permits compound of formula (II) or a salt thereof to be produced in high purity since the by-product can efficiently be removed by water washing.

Thus we provide a process for preparing a compound of formula (II) which comprises:
(a) reacting a compound of formula (III) with an activated derivative of 2-furoic acid as in an amount of at least 2 moles of the activated derivative per mole of compound of formula (III) to yield a compound of formula (IIA); 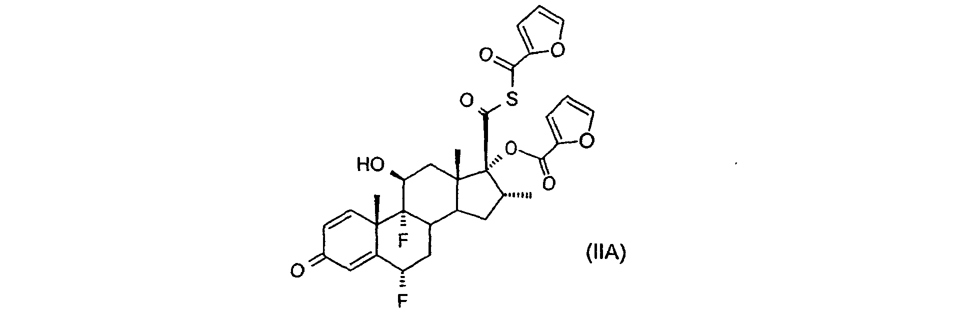 and
(b) removal of the sulphur-linked 2-furoyl moiety from compound of formula (IIA) by reaction of the product of step (a) with an organic primary or secondary amine base capable of forming a water soluble 2-furoyl amide.

In two particularly convenient embodiments of this process we also provide methods for the efficient purification of the end product which comprise either
(c1)when the product of step (b) is dissolved in a substantially water immiscible organic solvent, purifying the compound of formula (II) by washing out the amide by-product from step (b) with an aqueous wash, or
(c2) when the product of step (b) is dissolved in a water miscible solvent, purifying the compound of formula (II) by treating the product of step (b) with an aqueous medium so as to precipitate out pure compound of formula (II) or a salt thereof.

In step (a) preferably the activated derivative of 2-furoic acid may be an activated ester of 2-furoic acid, but is more preferably a 2-furoyl halide, especially 2-furoyl chloride. A suitable solvent for this reaction is ethylacetate or methylacetate (preferably methylacetate) (when step (c1) may be followed) or acetone (when step (c2) may be followed). Normally an organic base eg triethylamine will be present. In step (b) preferably the organic base is diethanolamine. The base may suitably be dissolved in a solvent eg methanol. Generally steps (a) and (b) will be performed at reduced temperature eg between 0 and 5°C. In step (c1) the aqueous wash may be water, however the use of brine results in higher yields and is therefore preferred. In step (c2) the aqueous medium is for example a dilute aqueous acid such as dilute HCI.

We also provide an alternative process for preparing a compound of formula (II) which comprises:
(a) reacting a compound of formula (III) with an activated derivative of 2-furoic acid in an amount of at least 2 moles of activated derivative per mole of compound of formula (III) to yield a compound of formula (IIA); and
(b) removal of the sulphur-linked 2-furoyl moiety from compound of formula (IIA) by reaction of the product of step (a) with a further mole of compound of formula (III) to give two moles of compound of formula (II).

In step (a) preferably the activated derivative of 2-furoic acid may be an activated ester of 2-furoic acid, but is more preferably a 2-furoyl halide, especially 2-furoyl chloride. A suitable solvent for his step is acetone. Normally an organic base eg triethylamine will be present. In step (b) a suitable solvent is DMF or dimethylacetamide. Normally an organic base eg triethylamine will be present. Generally steps (a) and (b) will be performed at reduced temperature eg between 0 and 5 °C. The product may be isolated by treatment with acid and washing with water.

This aforementioned process is very efficient in that it does not produce any furoylamide by-product (thus affording *inter alia* environmental advantages) since the excess mole of furoyl moiety is taken up by reaction with a further mole of compound of formula (II) to form an additional mole of compound of formula (II).

Further general conditions for the conversion of compound of formula (III) to compound of formula (II) in the two processes just described will be well known to persons skilled in the art.

According to a preferred set of conditions, however, we have found that the compound of formula (II) may advantageously be isolated in the form of a solid crystalline salt. The preferred salt is a salt formed with a base such as triethylamine, 2,4,6-trimethylpyridine, diisopropylethylamine or N-ethylpiperidine. Such salt forms of compound of formula (II) are more stable, more readily filtered and dried and can be isolated in higher purity than the free thioacid. The most preferred salt is the salt formed with diisopropylethylamine. The triethylamine salt is also of interest.

Compounds of formula (III) may be prepared in accordance with procedures described in GB 2088877B. Compounds of formula (III) may also be prepared by a process comprising the following steps:

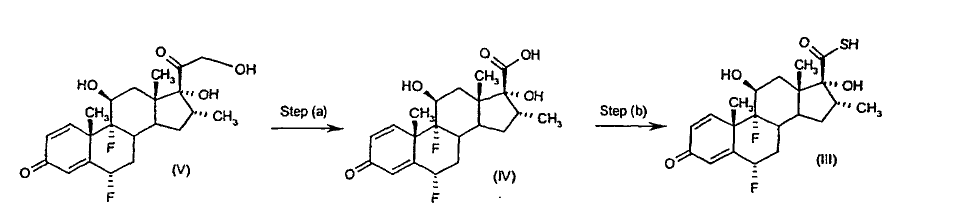

Step (a) comprises oxidation of a solution containing the compound of formula (V). Preferably, step (a) will be performed in the presence of a solvent comprising methanol, water, tetrahydrofuran, dioxan or diethylene glygol dimethylether. So as to enhance yield and throughput, preferred solvents are methanol, water or tetrahydrofuran, and more preferably are water or tetrahydrofuran, especially water and tetrahydrofuran as solvent. Dioxan and diethylene glygol dimethylether are also preferred solvents which may optionally (and preferably) be employed together with water. Preferably, the solvent will be present in an amount of between 3 and 10vol relative to the amount of the starting material (1wt.), more preferably between 4 and 6 vol., especially 5 vol. Preferably the oxidising agent is present in an amount of 1-9 molar equivalents relative to the amount of the starting material. For example, when a 50% w/w aqueous solution of periodic acid is employed, the oxidising agent may be present in an amount of between 1.1 and 10wt. relative to the amount of the starting material (1wt.), more preferably between 1.1 and 3wt., especially 1.3wt. Preferably, the oxidation step will comprise the use of a chemical oxidising agent. More preferably, the oxidising agent will be periodic acid or iodic acid or a salt thereof. Most preferably, the oxidising agent will be periodic acid or sodium periodate, especially periodic acid. Alternatively (or in addition), it will also be appreciated that the oxidation step may comprise any suitable oxidation reaction, eg one which utilises air and/or oxygen. When the oxidation reaction utilises air and/or oxygen, the solvent used in said reaction will preferably be methanol. Preferably, step (a) will involve incubating the reagents at room temperature or a little warmer, say around 25°C eg for 2 hours. The compound of formula (IV) may be isolated by recrystallisation from the reaction mixture by addition of an anti-solvent. A suitable anti-solvent for compound of formula (IV) is water. Surprisingly we have discovered that it is highly desirable to control the conditions under which the compound of formula (IV) is precipitated by addition of anti-solvent eg water. When the recrystallisation is performed using chilled water (eg water/ice mixture at a temperature of 0-5 °C) although better anti-solvent properties may be expected we have found that the crystalline product produced is very voluminous, resembles a soft gel and is very difficult to filter. Without being limited by theory we believe that this low density product contains a large amount of solvated solvent within the crystal lattice. By contrast when conditions of around 10°C or higher are used (eg around ambient temperature) a granular product of a sand like consistency which is very easily filtered is produced. Under these conditions, crystallisation typically commences after around 1 hour and is typically completed within a few hours (eg 2 hours). Without being limited by theory we believe that this granular product contains little or no solvated solvent within the crystal lattice.

Step (b) will typically comprise the addition of a reagent suitable for converting a carboxylic acid to a carbothioic acid eg using hydrogen sulphide gas together with a suitable coupling agent eg carbonyidiimidazole (CDI) in the presence of a suitable solvent eg dimethylformamide.

The advantages of the compositions of the invention may include the fact that they appears to demonstrate excellent anti-inflammatory properties, with predictable pharmacokinetic and pharmacodynamic behaviour, with an attractive side-effect profile, long duration of action, and is compatible with a convenient regime of treatment in human patients, in particular being amendable to once-per day dosing. Further advantages may include the fact that the compositions have desirable physical and chemical properties which allow for ready manufacture and storage.

The following non-limiting Examples illustrate the invention:

### EXAMPLES

### General

¹H-nmr spectra were recorded at 400 MHz and the chemical shifts are expressed in ppm relative to tetramethylsilane. The following abbreviations are used to describe the multiplicities of the signals: s (singlet), d (doublet), t (triplet), q (quartet), m (multiplet), dd (doublet of doublets), ddd (doublet of doublet of doublets), dt (doublet of triplets) and b (broad). Biotage refers to prepacked silica gel cartridges containing KP-Sil run on flash 12i chromatography module. LCMS was conducted on a Supelcosil LCABZ+PLUS column (3.3 cm x 4.6 mm ID) eluting with 0.1% HCO₂H and 0.01 M ammonium acetate in water (solvent A), and 0.05% HCO₂H 5% water in acetonitrile (solvent B), using the following elution gradient 0-0.7 min O%B, 0.7-4.2 min 100%B, 4.2-5.3 min O%B, 5.3-5.5 min O%B at a flow rate of 3 ml/min. The mass spectra were recorded on a Fisons VG Platform spectrometer using electrospray positive and negative mode (ES+ve and ES-ve).

### Intermediates

### Intermediate 1: 6α, 9α-Difluoro-17α-[(2-furanylcarbonyl)oxyl-11β-hydroxy-16α-methyl-3-oxo-androsta-1, 4-diene-17β-carbothioic acid diisopropylethylamine salt

A stirred suspension of 6α, 9α-difluoro-11β, 17α-dihydroxy-16α-methyl-3-oxo-androsta-1,4-diene-17β-carbothioic acid (prepared in accordance with the procedure described in GB 2088877B) (49.5g) in methylacetate (500ml) is treated with triethylamine (35ml) maintaining a reaction temperature in the range 0-5°C. 2-Furoyl chloride (25ml) is added and the mixture stirred at 0-5°C for 1 hour. A solution of diethanolamine (52.8g) in methanol (50ml) is added and the mixture stirred at 0-5°C for at least 2 hours. Dilute hydrochloric acid (approx 1M, 550ml) is added maintaining a reaction temperature below 15°C and the mixture stirred at 15°C. The organic phase is separated and the aqueous phase is back extracted with methyl acetate (2x250ml). All of the organic phases are combined, washed sequentially with brine (5 x 250ml) and treated with di-isopropylethylamine (30ml). The reaction mixture is concentrated by distillation at atmospheric pressure to an approximate volume of 250ml and cooled to 25-30°C (crystallisation of the desired product normally occurs during distillation/subsequent cooling). Tertiary butyl methyl ether (TBME) (500ml) is added, the slurry further cooled and aged at 0-5°C for at least 10 minutes. The product is filtered off, washed with chilled TBME (2x200ml) and dried under vacuum at approximately 40-50°C (75.3g, 98.7%). NMR (CDCl₃) δ: 7.54-7.46 (1H, m), 7.20-7.12 (1H. dd), 7.07-6.99 (1 H, dd), 6.48-6.41 (2H, m), 6.41-6.32 (1 H, dd), 5.51-5.28 (1 H, dddd ²J_{H-F} 50Hz), 4.45-4.33(1H, bd), 3.92-3.73 (3H, bm), 3.27-3.14 (2H, q), 2.64-2.12 (5H, m), 1.88-1.71 (2H, m), 1.58-1.15 (3H, s), 1.50-1.38 (15H, m), 1.32-1.23 (1 H, m), 1.23-1.15 (3H s), 1.09-0.99 (3H, d)

### Intermediate 2: 6α, 9α-Difluoro-17α-[(2-furanylcarbonyl)oxy]-11β-hydroxy-16α-methyl-3-oxo-androsta-1,4-diene-17β-carbothioic acid S-fluoromethyl ester Unsolvated Form 1

A mobile suspension of Intermediate 1 (12.61g, 19.8mmol) in ethyl acetate (230ml) and water (50ml) is treated with a phase transfer catalyst (benzyltributylammonium chloride, 10mol%), cooled to 3°C and treated with bromofluoromethane (1.10ml, 19.5mmol, 0.98 equivalents), washing in with prechilled (0°C) ethyl acetate (EtOAc) (20ml). The suspension is stirred overnight, allowing to warm to 17°C. The aqueous layer is separated and the organic phase is sequentially washed with 1M HCI (50ml), 1%w/v NaHCO₃ solution (3x50ml) and water (2x50ml). The ethylacetate solution is distilled at atmospheric pressure until the distillate reaches a temperature of approximately 73°C at which point toluene (150ml) is added. Distillation is continued at atmospheric pressure until all remaining EtOAc has been removed (approximate distillate temperature 103°C). The resultant suspension is cooled and aged at <10°C and filtered off. The bed is washed with toluene (2x30ml) and the product oven dried under vacuum at 60°C to constant weight to yield the title compound (8.77g, 82%) LCMS retention time 3.66min, m/z 539 MH⁺, NMR δ (CDCl₃) indudes 7.60 (1 H, m), 7.18 - 7.11 (2H, m), 6.52 (1H, dd, J 4.2Hz), 6.46 (1H, s), 6.41 (1H, dd,J 10, 2Hz), 5.95 and 5.82 (2H dd, J 51, 9Hz), 5.48 and 5.35 (1H, 2m), 4.48 (1H, m), 3.48 (1H, m), 1.55 (3H, s), 1.16 (3H, s), 1.06 (3H, d, J 7Hz).

### Pharmacological Activity

### In Vitro Pharmacological Activity

Pharmacological activity was assessed in a functional *in vitro* assay of glucocorticoid agonist activity which is generally predictive of anti-inflammatory or anti-allergic activity *in vivo.*
For the experiments in this section, compound of formula (I) was used as unsolvated Form 1 (Intermediate 2)

The functional assay was based on that described by K.P.Ray et al., Biochem J. (1997), 328, 707-715. A549 cells stably transfected with a reporter gene containing the NF-κB responsive elements from the ELAM gene promoter coupled to sPAP (secreted alkaline phosphatase) were treated with test compounds at appropriate doses for 1 hour at 37°C. The cells were then stimulated with tumour necrosis factor (TNF, 10ng/ml) for 16 hours, at which time the amount of alkaline phosphatase produced is measured by a standard colourimetric assay. Dose response curves were constructed from which EC₅₀ values were estimated.

In this test the compound of formula (I) showed an EC₅₀ value of <1nM.

The glucocorticoid receptor (GR) can function in at least two distinct mechanisms, by upregulating gene expression through the direct binding of GR to specific sequences in gene promotors, and by downregulating gene expression that is being driven by other transcription factors (such as NF_{K}B or AP-1) through their direct interaction with GR.

In a variant of the above method, to monitor these functions, two reporter plasmids have been generated and introduced separately into A549 human lung epithelial cells by transfection. The first cell line contains the firefly luciferase reporter gene under the control of a synthetic promoter that specifically responds to activation of the transcription factor NF_{K}B when stimulated with TNFα. The second cell line contains the renilla luciferase reporter gene under the control of a synthetic promotor that comprises 3 copies of the consensus glucocorticoid response element, and which responds to direct stimulation by glucocorticoids. Simultaneous measurement of transactivation and transrepression was conducted by mixing the two cell lines in a 1:1 ratio in 96 well plate (40,000 cells per well) and growing overnight at 37°C. Test compounds were dissolved in DMSO, and added to the cells at a final DMSO concentration of 0.7%. After incubation for 1h 0.5ng/ml TNFα (R&D Systems) was added and after a further 15 hours at 37°C, the levels of firefly and renilla luciferase were measured using the Packard Firelite kit following the manufacturers' directions. Dose response curves were constructed from which EC₅₀ values were determined.

| | Transactivation (GR) ED₅₀ (nM) | Transrepression (NF_{K}B) ED₅₀ (nM) |
|---|---|---|
| Compound of Formula (I) | 0.06 | 0.20 |
| Metabolite (X) | >250 | >1000 |
| Fluticasone propionate | 0.07 | 0.16 |

### In Vivo Pharmacological Activity

Pharmacological activity *in vivo* was assessed in an ovalbumin sensitised Brown Norway rat eosinophilia model. This model is designed to mimic allergen induced lung eosinophilia, a major component of lung inflammation in asthma.

For the experiments in this section, compound of formula (I) was used as unsolvated Form 1.

Compound of formula (I) produced dose dependant inhibition of lung eosinophilia in this model after dosing as an intra-tracheal (IT) suspension in saline 30 min prior to ovalbumin challenge. Significant inhibition is achieved after a single dose of 30µg of compound of formula (I) and the response was significantly (p=0.016) greater than that seen with an equivalent dose of fluticasone propionate in the same study (69% inhibition with compound of formula (I) vs 41% inhibition with fluticasone propionate).

In a rat model of thymus involution 3 daily IT doses of 100µg of compound (I) induced significantly smaller reductions in thymus weight (p= 0.004) than an equivalent dose of fluticasone propionate in the same study (67% reduction of thymus weight with compound (I) vs 78% reduction with fluticasone propionate).

Taken together these results indicate a superior therapeutic index for compound (I) compared to fluticasone propionate.

### In vitro metabolism in rat and human hepatocytes

Incubation of compound (I) with rat or human hepatocytes shows the compound to be metabolised in an identical manner to fluticasone propionate with the 17-β carboxylic acid (X) being the only significant metabolite produced. Investigation of the rate of appearance of this metabolite on incubation of compound (I) with human hepatocytes (37°C, 10µM drug concentration, hepatocytes from 3 subjects, 0.2 and 0.7 million cells/mL) shows compound (I) to be metabolised ca. 5-fold more rapidly than fluticasone propionate:-

| Subject number | Cell density (million cells/mL) | 17-β acid metabolite production (pmol/h) | |
|---|---|---|---|
| | | Compound (I) | Fluticasone propionate |
| 1 | 0.2 | 48.9 | 18.8 |
| 1 | 0.7 | 73.3 | 35.4 |
| 2 | 0.2 | 118 | 9.7 |
| 2 | 0.7 | 903 | 23.7 |
| 3 | 0.2 | 102 | 6.6 |
| 3 | 0.7 | 580 | 23.9 |

Median metabolite production 102-118 pmol/h for compound (I) and 18.8-23.0 pmol/h for fluticasone propionate.

### Pharmacokinetics after intravenous (IV) and oral dosing in rats

Compound (I) was dosed orally (0.1mg/kg) and IV (0.1 mg/kg) to male Wistar Han rats and pharmacokinetic parameters determined. Compound (I) showed negligible oral bioavailability (0.9%) and plasma clearance of 47.3 mUmin/kg, approaching liver blood flow (plasma clearance of fluticasone propionate = 45.2 mUmin/kg).

### Pharmacokinetics after intra-tracheal dry powder dosing in the pig.

Anaesthetised pigs (2) were dosed intra-tracheally with a homogenous mixture of compound (I) (1mg) and fluticasone propionate (1mg) as a dry powder blend in lactose (10% w/w). Serial blood samples were taken for up to 8h following dosing. Plasma levels of compound (I) and fluticasone propionate were determined following extraction and analysis using LC-MS/MS methodology, the lower limits of quantitation of the methods were 10 and 20pg/mL for compound (I) and fluticasone propionate respectively. Using these methods compound (I) was quantifiable up to 2 hours after dosing and fluticasone propionate was quantifiable up to 8 hours after dosing. Maximum plasma concentrations were observed for both compounds within 15min after dosing. Plasma half-life data obtained from IV dosing (0.1mg/kg) was used to calculate AUC (0-inf) values for compound (I). This compensates for the plasma profile of Compound (I) only being defined up to 2 hours after an IT dose and removes any bias due to limited data between compound (I) and fluticasone propionate.

Cₘₐₓ and AUC (0-inf) values show markedly reduced systemic exposure to compound (I) compared to fluticasone propionate:-

| | Cmax (pg/mL) | | AUC (0-inf) (hr.pg/mL) | |
|---|---|---|---|---|
| | Pig 1 | Pig 2 | Pig 1 | Pig 2 |
| Compound of Formula (I) | 117 | 81 | 254 | 221 |
| Fluticasone propionate | 277 | 218 | 455 | 495 |

The pharmacokinetic parameters for both compound (I) and fluticasone propionate were the same in the anaesthetised pig following intravenous administration of a mixture of the two compounds at 0.1 mg/kg. The clearance of these two glucocorticoids is similar is this experimental pig model.

### Example 1: Dry powder composition containing 6α, 9α-Difluoro-17α-[(2-furanylcarbonyl)oxy]-11β-hydroxy-16α-methyl-3-oxo-androsta-1,4-diene-17β-carbothioic acid S-fluoromethyl ester, Unsolvated Form 1, and a long acting muscarinic receptor antagonist

A dry powder formulation may be prepared as follows:

| | |
|---|---|
| 6α, 9α-Difluoro-17α-[(2-furanylcarbonyl)oxy]-11β-hydroxy-16α-methyhl-3-oxo-androsta-1,4-diene-17β-carbothioic acid S-fluoromethyl ester, unsolvated Form 1 (prepared according to Intermediate 2 and micronised to a MMD of 3µm) | 0.20mg |
| Tiotropium bromide (micronised to a MMD of 3µm) | 0.01 mg |
| milled lactose (wherein not greater than 85% of particles have a MMD of 60-90µm, and not less than 15% of particles have a MMD of less than 15µm) | 12mg |

A peelable blister strip containing 60 blisters each filled with a formulation as just described may be prepared.

### Example 2: Aerosol formulation containing 6α, 9α-Difluoro-17α-[(2-furanylcarbonyl)oxy]-11β-hydroxy-16α-methyl-3-oxo-androsta-1,4-diene-17β-carbothioic acid S-fluoromethyl ester, Unsolvated Form 1, and a long acting muscarinic receptor antagonist

An aluminium canister may be filled with a formulation as follows:

| | |
|---|---|
| 6α, 9α-Difluoro-17α-[(2-furanylcarbonyl)oxy]-11β-hydroxy-16α-methyl-3-oxo-androsta-1,4-diene-17β-carbothioic acid S-fluoromethyl ester, Unsolvated Form 1 (prepared according to Intermediate 2) and micronised to a MMD of 3µm) | 250 µg |
| Tiotropium bromide (micronised to a MMD of 3µm) | 20 µg |
| 1,1,1,2-tetrafluoroethane (amounts per actuation) | to 50µl |

in a total amount suitable for 120 actuations and the canister may be fitted with a metering valve adapted to dispense 50 µl per actuation.

Inhalers of the invention will now be described, by way of example only, with reference to, and as shown in, the accompanying drawings in which:
Figures 1 to 4 relate to a dry powder inhalers;
Figure 5 relates to a metered dose inhaler;
Figure 6 relates to a nasal inhaler.

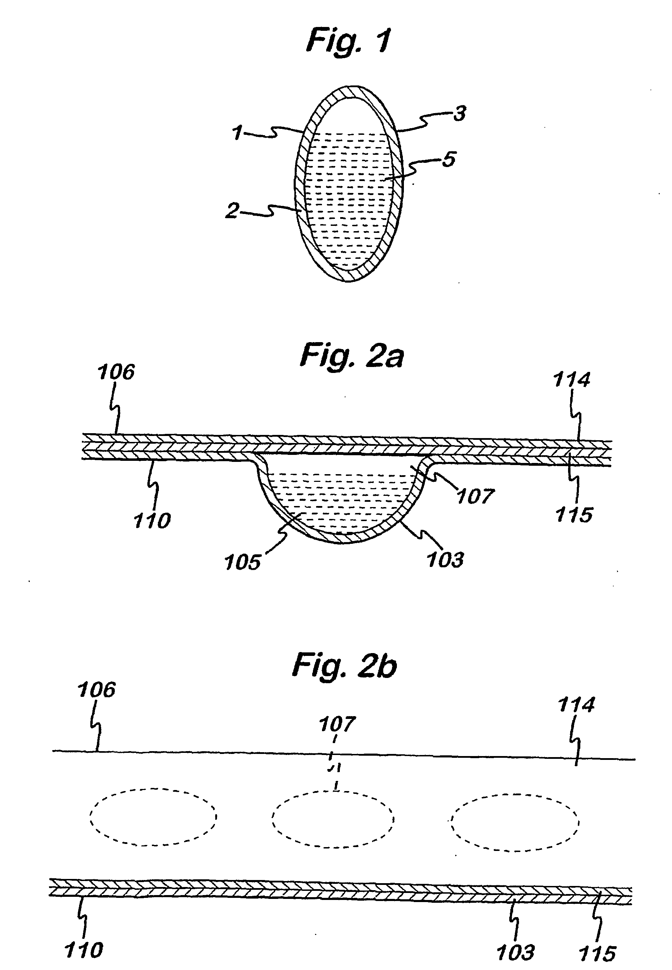

Figure 1 shows a suitable medicament carrier in the form of a capsule.

Figure 2a is a cross-sectional side elevation of a suitable elongate medicament blister strip.

Figure 2b is a top perspective of the medicament blister strip illustrated in Figure 2a.

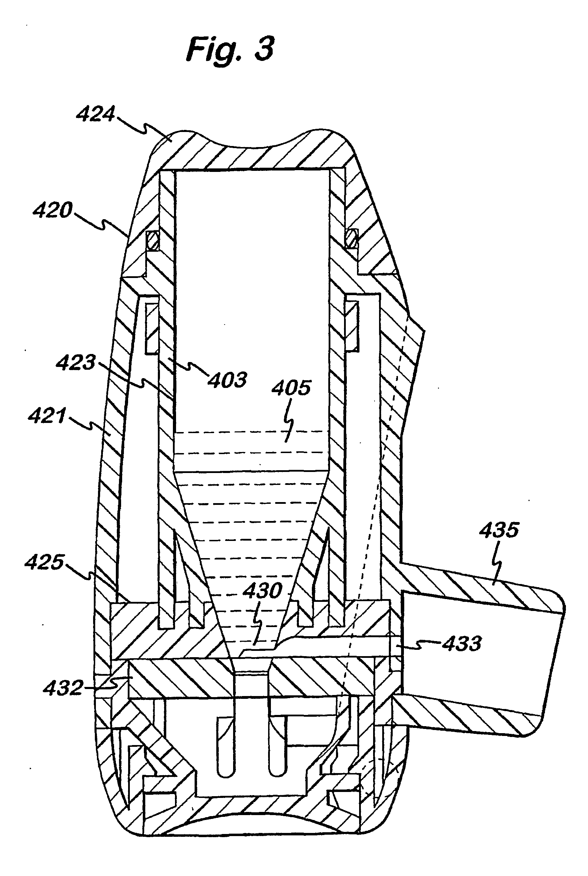

Figure 3 shows a cross-sectional dry powder inhaler (DPI) comprising a powder reservoir.

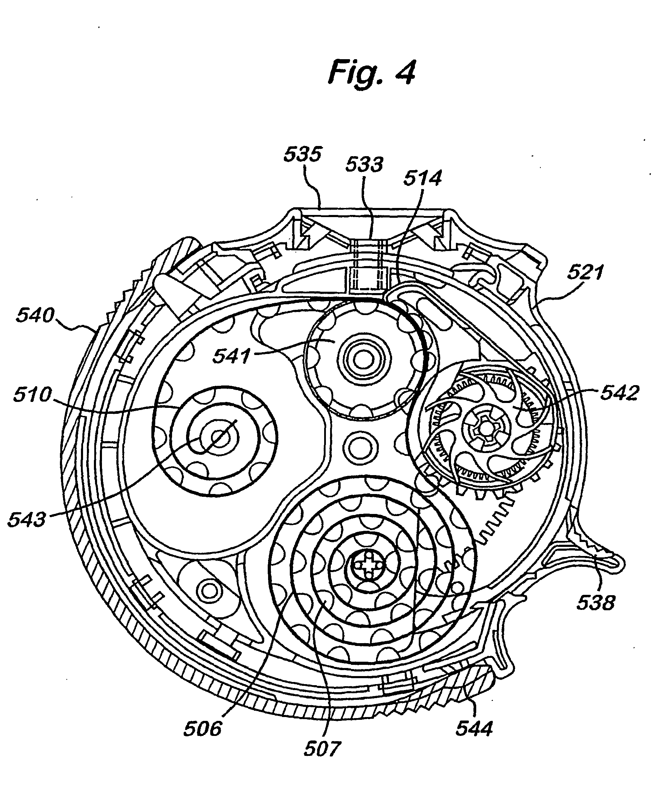

Figure 4 shows a cross-sectional dry powder inhaler (DPI) comprising an elongate medicament blister strip.

The medicament carrier in Figure 1 is in the form of a capsule 1 comprising a wall 2 enclosing medicament powder 5. Medicament powder 5 is released on piercing the wall 2 of capsule 1 and may be inhaled by a patient.

Figure 2a shows a sectional side-elevation of a single blister strip 106 comprising a pocket 107, containing dry powder 105, base 110 and lid comprising laminates 114, 115. The lid is composed of a metallic foil laminate 114 bound to a plastic laminate 115. In the diagram, the lid 114, 115 is hermetically sealed to base 110 by appropriate means (e.g. adhesion, welding). Base 110 comprises an organic polymeric plastic 103. A top perspective view of the blister strip 106 showing pockets 107 is illustrated in Figure 2b. Laminated lid 114, 115 is sealed to base 110.

Figure 3 shows a sectional view of a dry powder inhaler 420 for dispensing medicament in accord with the present invention. The inhaler 420 comprises a body 421 which defines a reservoir 423 and a reservoir cover 424. The reservoir contains a supply of medicament in dry powder form 405. The walls 423 of the reservoir, defined by the body 421, are comprised of a plastic material 403. Base 425 and body 421 define an aperture 430 through which powder 405 can pass from the reservoir to the dosing member 432. Powder 405 is guided by the walls 423 of the reservoir, which form a hopper, to the dosing member 432. Extending laterally from the lower end of the main body 421 is mouthpiece 435, through which the patient inhales via passage 433. If the device were intended for nasal inhalation this would be replaced by a nosepiece.

Figure 4 shows a simplified cross-sectional plan view of a dry powder inhaler comprising an elongate medicament carrier suitable for dispensing medicament in accord with the present invention. The inhaler 540 dispenses unit doses of medicament powder from a medicament blister strip 506. The inhaler is comprised of an outer casing 544 enclosing a medicament strip 506 within body 521. The medicament strip may be, for example, any of those described in Figures 2a to 2b above. The patient uses the inhaler by holding the device to his mouth, depressing lever 538, and inhaling through mouthpiece 535. Depression of lever 538 activates the internal mechanism of the inhaler, such that the lid 514 and base 510 sheets of coiled medicament blister strip 506 are separated at index wheel 541 by use of contracting wheel 542 and base wheel 543. A unit dose of powdered medicament within blister pocket 507 is released and may be inhaled by the patient through exit port 533 and mouthpiece 535.

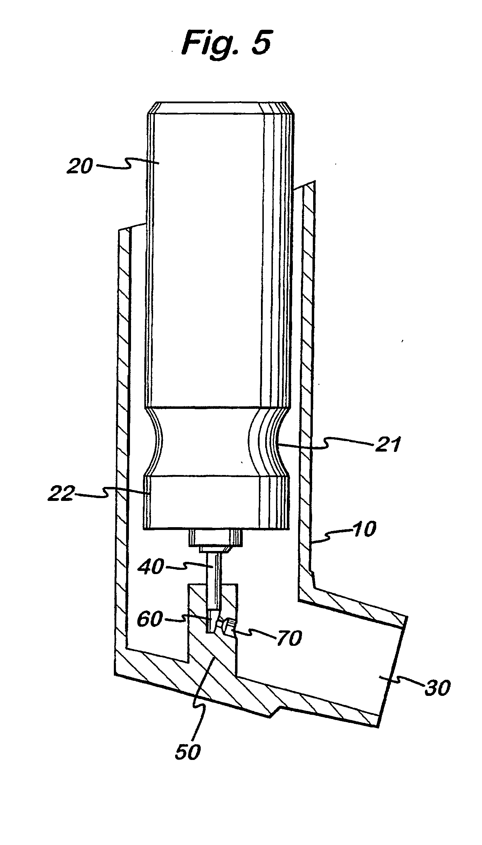

Figure 5 is a schematic representation of a section through a standard metered dose inhalation device.

The standard metered dose inhaler shown in Fig 1 comprises a housing 10 in which an aerosol canister 20 can be located. The housing is open at one end (which will hereinafter be considered to be the top of the device for convenience of description) and is closed at the other. An outlet 30 leads laterally from the closed end of the housing 10. In the embodiment illustrated, the outlet 30 is in the form of a mouthpiece intended for insertion into the mouth of the patient but it may, if desired, be designed as a nozzle for insertion into the patient's nostril.

The aerosol canister 20, comprising a neck region 21 and ferrule 22, has an outlet valve stem 40 at one end. This valve member can be depressed to release a measured dose from the aerosol canister or, alternatively, the valve stem 40 can be fixed and the main body of the canister can be moved relative to the valve member to release the dose.

As shown in Figure 5, the aerosol canister 20 is located in the housing 10 so that one end protrudes from its open top, the canister being positioned such that the neck 21 and valve ferrule 22 are enclosed within housing 10. Spacer ribs (not shown) may be provided inside the housing to hold the external surface of the canister 20 spaced from the internal surface of the housing 10. A support 50 is provided at the lower end of the housing 10 and has a passage 60 in which the valve stem 40 of the aerosol canister 20 can be located and supported. A second passage 70 is provided in the support 50 and is directed towards the interior of the outlet 30. Thus, when the parts are in the positions shown in Figure 1, the protruding portion of the aerosol canister 20 can be depressed to move the canister relative to the valve stem 40 to open the valve and a dose of medicament contained in the aerosol will be discharged through the passage 70 and into the outlet 30 from which it can be inhaled by a patient. One dose will be released from the aerosol canister each time it is fully depressed.

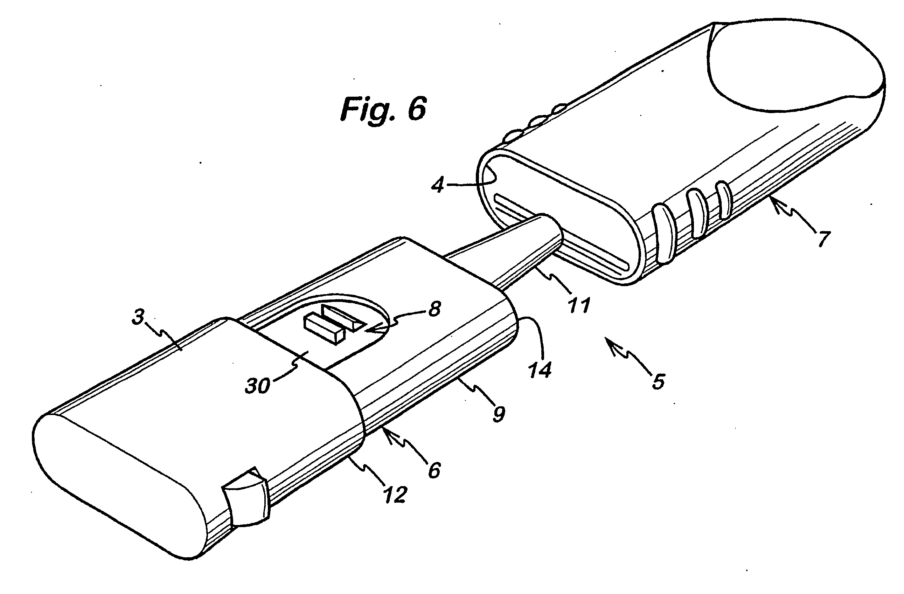

Fig. 6 shows an exploded view of a nasal inhaler suitable for use in accord with the invention;

With reference to Figure 6 there is shown a nasal inhaler device 5 comprising a body 6 a container 3 and a nasal pump 8. The device further comprises a protective end cap 7 having an inner surface 4 for engagement with the body 6 to protect the dispensing nozzle 11.

The body 6 is made from a plastic material and defines a housing 9 and a dispensing nozzle 11. The housing 9 defines a cavity formed by a side wall and a first end wall and a second end wall 14. The dispensing nozzle 11 is connected to and extends away from the second end wall 14 and has an external tapering form.

The dispensing nozzle 11 has a longitudinally extending orifice defined by an outlet tube extending towards the cavity 10. An annular abutment is formed within the orifice part way along the outlet tube. The annular abutment defines a small aperture through which fluid can flow in use.

The nasal pump 8 comprises a hollow casing 30 defining a reservoir containing several doses of the fluid nasal formulation to be dispensed and a plunger slidably engaged within the hollow casing 30. One detailed example of a suitable nasal pump is described in US-A-4,964,069 incorporated herein by reference.

Throughout the specification and the claims which follow, unless the context requires otherwise, the word 'comprise', and variations such as 'comprises' and 'comprising', will be understood to imply the inclusion of a stated integer or step or group of integers but not to the exclusion of any other integer or step or group of integers or steps.

The patents and patent applications described in this application are herein incorporated by reference.

## Claims

1. A pharmaceutical composition comprising a compound of formula (I), 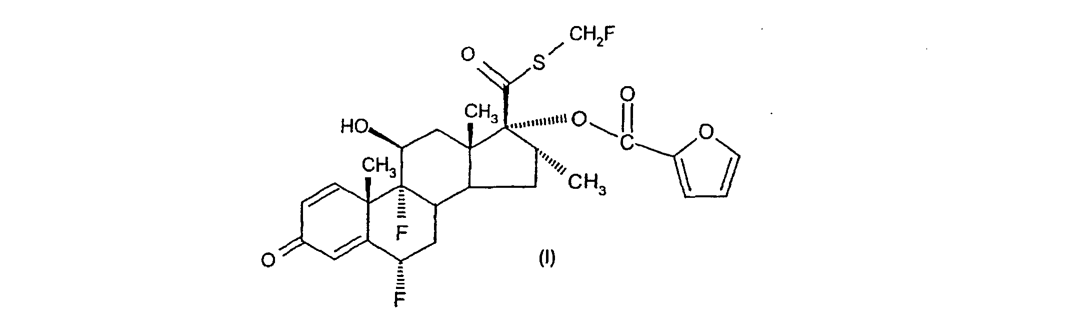 or a solvate thereof, in combination with a muscarinic receptor antagonist.

2. A pharmaceutical composition according to claim 1 wherein the muscarinic receptor antagonist is selective for the M1 and M3 receptors over the M2 receptor.

3. A pharmaceutical composition according to claim 1 wherein the muscarinic receptor antagonist is ipratropium or a salt thereof or oxitropium or a salt thereof.

4. A pharmaceutical composition according to claim 1 or claim 2 wherein the muscarinic receptor antagonist has a duration of action sufficient to provide a therapeutic effect when dosed once or twice per day.

5. A pharmaceutical composition according to claim 4 wherein the muscarinic receptor antagonist is tiotropium or a salt thereof.

6. A pharmaceutical composition according to claim 1 wherein the muscarinic receptor antagonist is a compound of formula (A): 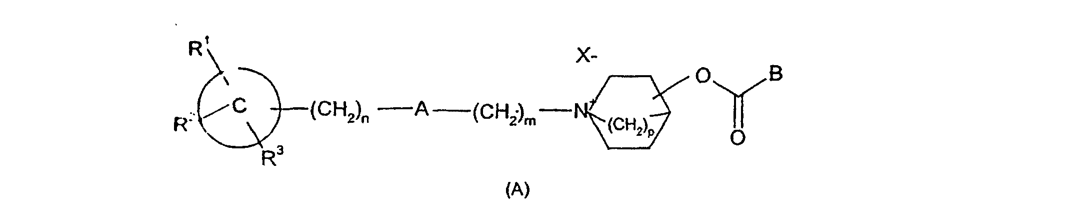 wherein: C is a phenyl ring, a C₄ to C₉ heteroaromatic group containing one or more heteroatoms (preferably selected from nitrogen, oxygen and sulphur atoms), or a naphthalenyl, 5,6,7,8-tetrahydronaphthalenyl or biphenyl group;
R¹, R² and R³ each independently represent a hydrogen or halogen atom, or a hydroxy group, or a phenyl, -OR⁴, -SR⁴, -NR⁴R⁵, -NHCOR⁴, -CONR⁴R⁵,-CN, - NO₂ -COOR⁴ or -CF₃ group, or a straight or branched lower alkyl group which may optionally be substituted, for example, with a hydroxy or alkoxy group, wherein R⁴ and R⁵ each independently represent a hydrogen atom, straight or branched lower alkyl group, or together form an alicyclic ring; or R¹ and R² together form an aromatic, alicyclic or heterocyclic ring;
n is an integer from 0 to 4;
A represents a -CH₂-, -CH=CR⁶-, -CR⁶=CH-, -CR⁶R⁷-, -CO-, -O-, -S-, -S(O)-, SO₂ or -NR⁶- group, wherein R⁶ and R⁷ each independently represent a hydrogen atom, straight or branched lower alkyl group, or R⁶ and R⁷ together form an alicyclic ring;
m is an integer from 0 to 8; provided that when m = 0, A is not -CH₂-;
p is an integer from 1 to 2 and the substitution in the azoniabicyclic ring may be in the 2, 3 or 4 position including all possible configurations of the asymmetric carbons;
B represents a group of formula (i) or (ii): 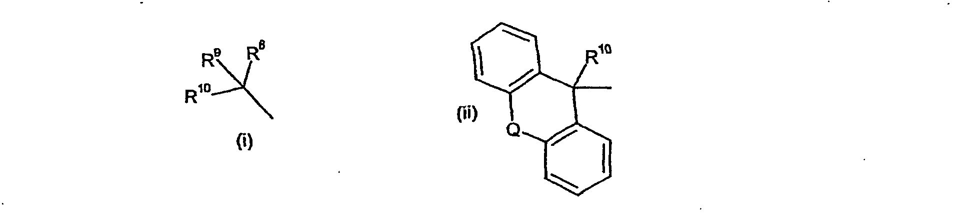 wherein R¹⁰ represents a hydrogen atom, a hydroxy or methyl group; and R⁸ and R⁹ each independently represents one of the following 5 moieties: 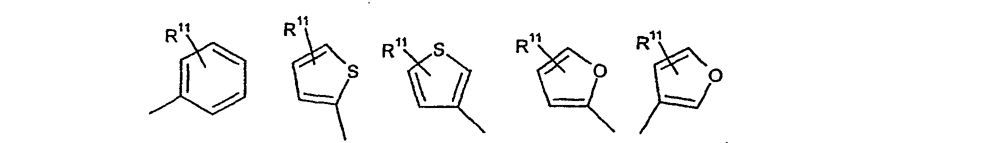 wherein R¹¹ represents a hydrogen or halogen atom, or a straight or branched lower alkyl group and Q represents a single bond, -CH₂-, -CH₂-CH₂-, -O-, -O-. CH₂-, -S-, -S-CH₂- or -CH=CH-, and when (i) or (ii) contain a chiral centre they may represent either configuration;
X represent a pharmaceutically acceptable anion of a mono or polyvalent acid.

7. A pharmaceutical composition according to daim 6 wherein where © represents a phenyl, pyrrolyl, or thienyl group; R¹, R² and R³ each independently represent a hydrogen atom, a hydroxy group or a halogen atom; n = 0 or 1; m is an integer 1, 2 or 3; A represents a -CH₂-, -CH=CH- or -0- group; p = 2 and the substituent group -OC(O)B attached to the azoniabicyclo[2.2.2]octane is at the 3 position having the (R) configuration; the -OC(O)B group is diphenylacetoxy, 2-hydroxy-2,2-diphenyl-acetoxy, 2,2-diphenylpropionyloxy, 2-hydroxy-2-phenyl-2-thien-2-yl-acetoxy, 2-furan-2-yl-2-hydroxy-2-phenylacetoxy, 2,2-dithien-2-ylacetoxy, 2-hydroxy-2,2-di-thien-2-ylacetoxy, 2-hydroxy-2,2-di-thien-3-ylacetoxy, 9-hydroxy-9[H]-fluorene-9-carbonyloxy, 9-methyl-9[H]-fluorene-9-carbonyloxy, 9[H]-xanthene-9-carbonyloxy, 9-hydroxy-9[H]-xanthene-9-carbonyloxy or 9-methyl-9[H]-xanthene-9-carbonyloxy; and the azoniabicyclo group is substituted on the nitrogen atom with a 3-phenoxypropyl, 2-phenoxypropyl, 3-phenylallyl, phenethyl, 4-phenylbutyl, 3-phenylpropyl, 3-[2-hydroxyphenoxy]propyl, 3-[4-flurophenoxy]propyl, 2-benzyloxyethyl, 3-pyrrol-1-ylpropyl, 2-thien-2-ylethyl or 3-thien-2-ylpropyl group.

8. A pharmaceutical composition according to any one of claims 1 to 8 wherein the compound of formula (I) or a solvate thereof and the muscarinic receptor antagonist are both present in particulate form.

9. A pharmaceutical composition according to claim 8 further comprising a particulate carrier.

10. A pharmaceutical composition according to claim 9 wherein the carrier is lactose.

11. A pharmaceutical composition according to any one of claims 1 to 8 further comprising a liquefied propellant gas.

12. A pharmaceutical composition according to any one of claims 1 to 11 wherein the compound of formula (I) is in unsolvated form.

13. A pharmceutical composition according to claim 12 wherein the compound of formula (I) is in unsolvated form as polymorph Form 1.

14. A pharmceutical composition according to any one of claims 1 to 13 further comprising a long-acting β₂-adrenoreceptor agonist.

15. A pharmaceutical composition according to any one of claims 1 to 14 adapted for administration by inhalation.

16. A pharmaceutical composition according to claim 15 for use in the treatment of inflammatory and allergic disorders of the respiratory tract.

17. Use of a pharmaceutical composition according to any one of claims 1 to 14 for the preparation of a medicament for the treatment of an inflammatory disorder of the respiratory tract which comprises administration by inhalation.

18. Use of the pharmaceutical composition according to claim 17 wherein the inflammatory disorder of the respiratory tract is COPD or asthma.

19. A formulation according to any one of claims 1 to 16 for use as a medicament in human or veterinary medicine in the treatment of patients with inflammatory and/or allergic condition for treatment once-per-day.

20. The use of a formulation according to any one of claims 1 to 16 for the manufacture of a medicament for the treatment of a patient with an inflammatory and/or allergic condition

21. An inhaler containing a plurality of doses of a pharmaceutical formulation comprising a compound of formula (I) 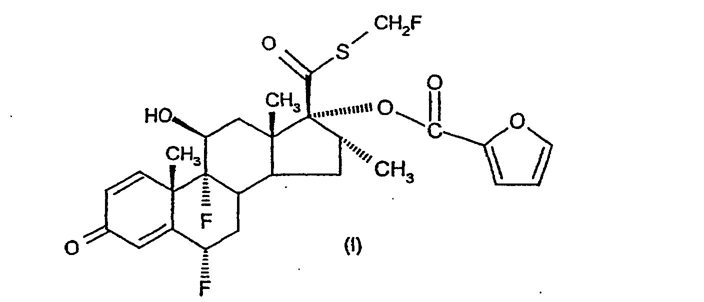 or a solvate thereof, in combination with a long-acting muscarinic receptor antagonist, which formulation has a therapeutically useful effect in the treatment of inflammatory disorders of the respiratory tract over a period of 24 hours or more, and which doses are suitable for once-per-day administration of the formulation by inhalation.

22. An inhaler according to claim 21 wherein the compound of formula (I) or a solvate thereof and the long-acting muscarinic receptor antagonist are both present in particulate form.

23. An inhaler according to claim 22 wherein the formulation further comprises a particulate carrier.

24. An inhaler according to claim 23 wherein the carrier is lactose.

25. An inhaler according to claim 21 or 22 wherein the formulation further comprises a liquefied propellant gas.

26. An inhaler containing a plurality of doses of a pharmaceutical formulation comprising a particulate compound of formula (I) 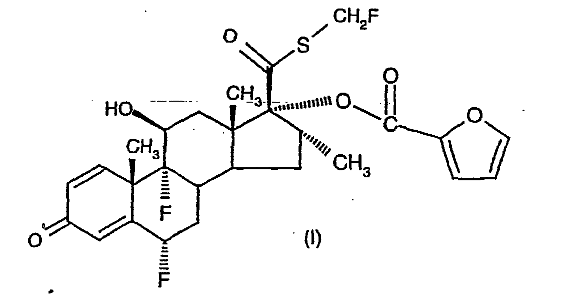 or a solvate thereof, a particulate long-acting muscarinic receptor antagonist and a carrier, each drug being present in an amount adequate to provide a therapeutically useful effect in the treatment of inflammatory disorders of the respiratory tract over a period of 24 hours or more following once-per-day dosing by inhalation.

27. An inhaler according to any one of claims 21 to 26 wherein wherein the inflammatory disorder of the respiratory tract is asthma or COPD.

## Patentansprüche

1. Pharmazeutische Zusammensetzung, die eine Verbindung der Formel (I): 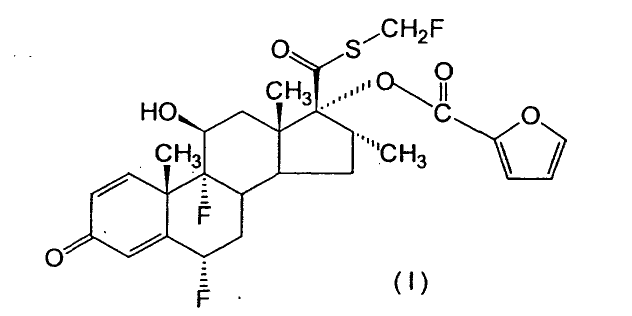 oder ein Solvat davon in Kombination mit einem Muscarinrezeptor-Antagonisten umfasst.

2. Pharmazeutische Zusammensetzung gemäss Anspruch 1, worin der Muscarinrezeptor-Antagonist selektiv für die M1- und M3-Rezeptoren gegenüber dem M2-Rezeptor ist.

3. Pharmazeutische Zusammensetzung gemäss Anspruch 1, worin der Muscarinrezeptor-Antagonist Ipratropium oder ein Salz davon oder Oxitropium oder ein Salz davon ist.

4. Pharmazeutische Zusammensetzung gemäss Anspruch 1 oder 2, worin der Muscarinrezeptor-Antagonist eine Wirkungsdauer hat, die ausreichend zur Bereitstellung einer therapeutischen Wirkung bei ein- oder zweimaliger Dosierung pro Tag ist.

5. Pharmazeutische Zusammensetzung gemäss Anspruch 4, worin der Muscarinrezeptor-Antagonist Tiotropium oder ein Salz davon ist.

6. Pharmazeutische Zusammensetzung gemäss Anspruch 1, worin der Muscarinrezeptor-Antagonist eine Verbindung der Formel (A) ist: 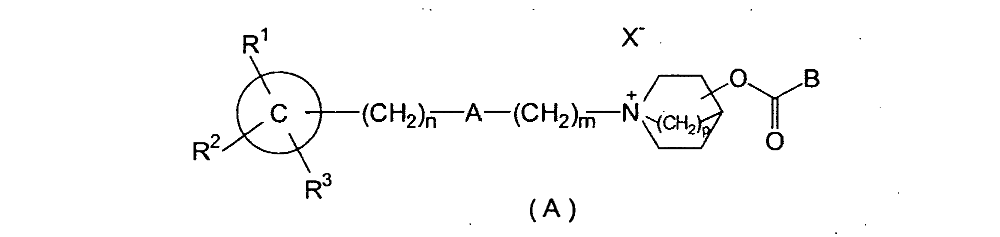 worin:
C ein Phenylring, eine C₄₋₉-heteroaromatische Gruppe, die ein oder mehrere Heteroatome enthält (bevorzugt aus Stickstoff-, Sauerstoff- und Schwefelatomen ausgewählt), oder eine Naphthalenyl-, 5,6,7,8-Tetrahydronaphthalenyl- oder Biphenylgruppe ist;
R¹, R² und R³ jeweils unabhängig ein Wasserstoff- oder Halogenatom oder eine Hydroxygruppe oder eine Phenyl-, -OR⁴-, -SR⁴-, -NR⁴R⁵-, -NHCOR⁴-, -CONR⁴R⁵-, -CN-, -NO₂-, -COOR⁴- oder -CF₃-Gruppe oder eine lineare oder verzweigte Niederalkylgruppe, die gegebenenfalls mit z.B. einer Hydroxy- oder Alkoxygruppe substituiert sein kann, darstellen, worin R⁴ und R⁵ jeweils unabhängig ein Wasserstoffatom oder eine lineare oder verzweigte Niederalkylgruppe darstellen oder zusammen einen alicyclischen Ring bilden; oder R¹ und R² zusammen einen aromatischen, alicyclischen oder heterocyclischen Ring bilden;
n eine ganze Zahl von 0 bis 4 ist;
A eine -CH₂-, -CH=CR⁶-, -CR⁶=CH-, -CR⁶R⁷-, -CO-, -O-, -S-, -S(O)-, SO₂- oder -NR⁶-Gruppe darstellt, worin R⁶ und R⁷ jeweils unabhängig ein Wasserstoffatom oder eine lineare oder verzweigte Niederalkylgruppe darstellen oder R⁶ und R⁷ zusammen einen alicyclischen Ring bilden;
m eine ganze Zahl von 0 bis 8 ist; mit der Massgabe, dass dann, wenn m = 0 ist, A nicht -CH₂- ist;
p eine ganze Zahl von 1 bis 2 ist und die Substitution im azoniabicyclischen Ring in der 2-, 3- oder 4-Position sein kann, einschliesslich aller möglichen Konfigurationen der asymmetrischen Kohlenstoffatome;
B eine Gruppe der Formel (i) oder (ii) darstellt: 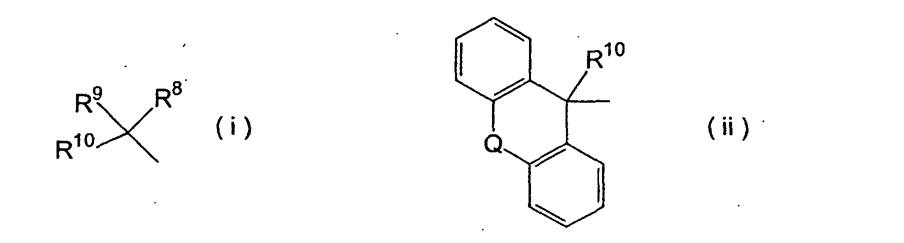
worin R¹⁰ ein Wasserstoffatom oder eine Hydroxy- oder Methylgruppe darstellt; und R⁸ und R⁹ jeweils unabhängig eine der folgenden 5 Einheiten darstellen: 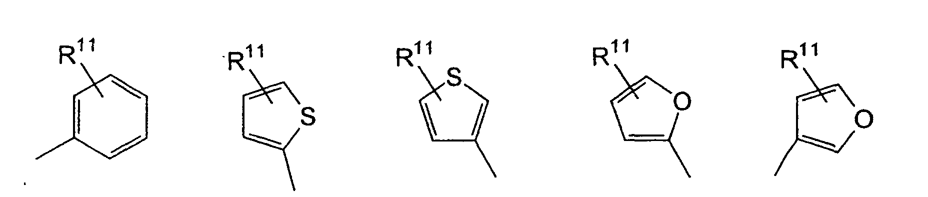 worin R¹¹ ein Wasserstoff- oder Halogenatom oder eine lineare oder verzweigte Niederalkylgruppe darstellt und Q eine Einfachbindung, -CH₂-, -CH₂-CH₂-, -O-, -O-CH₂-, -S-, -S-CH₂- oder -CH=CH- darstellt, und worin dann, wenn (i) oder (ii) ein chirales Zentrum enthält, sie jede Konfiguration darstellen können;
X ein pharmazeutisch akzeptables Anion einer ein- oder mehrwertigen Säure darstellt.

7. Pharmazeutische Zusammensetzung gemäss Anspruch 6, worin © eine Phenyl-, Pyrrolyl- oder Thienylgruppe darstellt; R¹, R² und R³ jeweils unabhängig ein Wasserstoffatom, eine Hydroxygruppe oder ein Halogenatom darstellen; n = 0 oder 1 ist; m eine ganze Zahl aus 1, 2 oder 3 ist; A eine -CH₂-, -CH=CH- oder -O-Gruppe darstellt; p = 2 ist und die an das Azoniabicyclo[2.2.2]octan gebundene Substituentengruppe -OC(O)B in der 3-Position mit der (R)-Konfiguration ist; die -OC(O)B-Gruppe Diphenylacetoxy, 2-Hydroxy-2,2-diphenyl-acetoxy, 2,2-Diphenylpropionyloxy, 2-Hydroxy-2-phenyl-2-thien-2-yl-acetoxy, 2-Furan-2-yl-2-hydroxy-2-phenylacetoxy, 2,2-Dithien-2-ylacetoxy, 2-Hydroxy-2,2-di-thien-2-ylacetoxy, 2-Hydroxy-2,2-di-thien-3-ylacetoxy, 9-Hydroxy-9[H]-fluoren-9-carbonyloxy, 9-Methyl-9[H]-fluoren-9-carbonyloxy, 9[H]-Xanthen-9-carbonyloxy, 9-Hydroxy-9[H]-xanthen-9-carbonyloxy oder 9-Methyl-9[H]-xanthen-9-carbonyloxy ist; und die Azoniabicyclogruppe am Stickstoffatom mit einer 3-Phenoxypropyl-, 2-Phenoxypropyl-, 3-Phenylallyl-, Phenethyl-, 4-Phenylbutyl-, 3-Phenylpropyl-, 3-[2-Hydroxyphenoxy]propyl-, 3-[4-Fluorphenoxy]propyl-, 2-Benzyloxyethyl-, 3-Pyrrol-1-ylpropyl-, 2-Thien-2-ylethyl- oder 3-Thien-2-ylpropyl-Gruppe substituiert ist.

8. Pharmazeutische Zusammensetzung gemäss einem der Ansprüche 1 bis 8, worin die Verbindung der Formel (I) oder ein Solvat davon und der Muscarinrezeptor-Antagonist beide in Teilchenform vorhanden sind.

9. Pharmazeutische Zusammensetzung gemäss Anspruch 8, die ferner einen teilchenförmigen Träger umfasst.

10. Pharmazeutische Zusammensetzung gemäss Anspruch 9, worin der Träger Lactose ist.

11. Pharmazeutische Zusammensetzung gemäss einem der Ansprüche 1 bis 8, die ferner ein verflüssigtes Treibmittelgas umfasst.

12. Pharmazeutische Zusammensetzung gemäss einem der Ansprüche 1 bis 11, worin die Verbindung der Formel (I) in unsolvatisierter Form ist.

13. Pharmazeutische Zusammensetzung gemäss Anspruch 12, worin die Verbindung der Formel (I) in unsolvatisierter Form als polymorphe Form 1 ist.

14. Pharmazeutische Zusammensetzung gemäss einem der Ansprüche 1 bis 13, die ferner einen langwirkenden β₂-Adrenorezeptor-Agonisten umfasst.

15. Pharmazeutische Zusammensetzung gemäss einem der Ansprüche 1 bis 14, die zur Verabreichung durch Inhalation angepasst ist.

16. Pharmazeutische Zusammensetzung gemäss Anspruch 15 zur Verwendung in der Behandlung von inflammatorischen und allergischen Störungen der Atemwege.

17. Verwendung einer pharmazeutischen Zusammensetzung gemäss einem der Ansprüche 1 bis 14 zur Herstellung eines Medikaments zur Behandlung einer inflammatorischen Störung der Atemwege, welche die Verabreichung durch Inhalation umfasst.

18. Verwendung der pharmazeutischen Zusammensetzung gemäss Anspruch 17, worin die inflammatorische Störung der Atemwege COPD oder Asthma ist.

19. Formulierung gemäss einem der Ansprüche 1 bis 16 zur Verwendung als Medikament in der Human- oder Veterinärmedizin in der Behandlung von Patienten mit einem inflammatorischen und/oder allergischen Zustand zur Behandlung einmal täglich.

20. Verwendung einer Formulierung gemäss einem der Ansprüche 1 bis 16 zur Herstellung eines Medikaments zur Behandlung eines Patienten mit einem inflammatorischen und/oder allergischen Zustand.

21. Inhalator, der eine Mehrzahl von Dosen einer pharmazeutischen Formulierung enthält, die eine Verbindung der Formel (I): 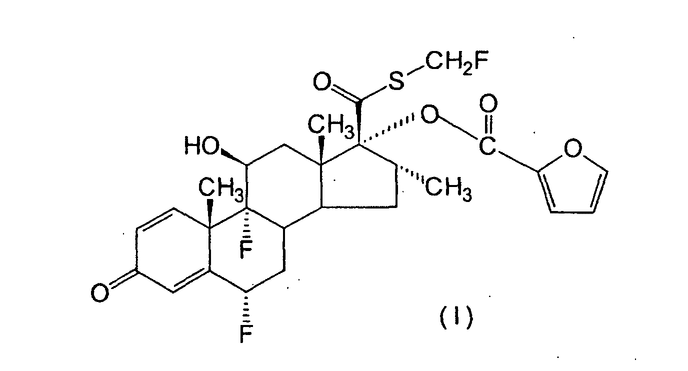 oder ein Solvat davon in Kombination mit einem langwirkenden Muscarinrezeptor-Antagonisten umfasst, wobei die Formulierung eine therapeutisch nützliche Wirkung in der Behandlung von inflammatorischen Störungen der Atemwege über einen Zeitraum von 24 Stunden oder mehr hat und wobei die Dosen zur einmal täglichen Verabreichung der Formulierung durch Inhalation geeignet sind.

22. Inhalator gemäss Anspruch 21, worin die Verbindung der Formel (I) oder ein Solvat davon und der langwirkende Muscarinrezeptor-Antagonist beide in Teilchenform vorhanden sind.

23. Inhalator gemäss Anspruch 22, worin die Formulierung ferner einen teilchenförmigen Träger umfasst.

24. Inhalator gemäss Anspruch 23, worin der Träger Lactose ist.

25. Inhalator gemäss Anspruch 21 oder 22, worin die Formulierung ferner ein verflüssigtes Treibmittelgas umfasst.

26. Inhalator, der eine Mehrzahl von Dosen einer pharmazeutischen Formulierung enthält, die eine teilchenförmige Verbindung der Formel (I): 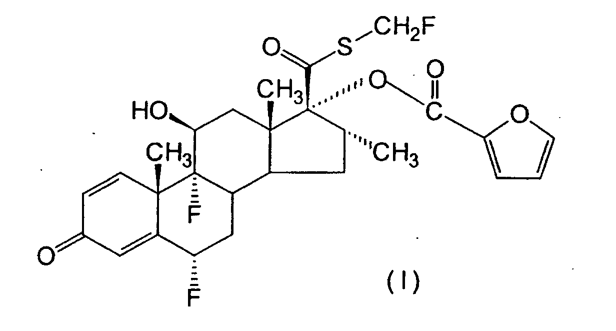 oder ein Solvat davon, einen teilchenförmigen, langwirkenden Muscarinrezeptor-Antagonisten und einen Träger umfasst, wobei jeder Wirkstoff in einer angemessenen Menge vorhanden ist, um eine therapeutisch nützliche Wirkung in der Behandlung von inflammatorischen Störungen der Atemwege über einen Zeitraum von 24 Stunden oder mehr nach einer einmal täglichen Dosierung durch Inhalation bereitzustellen.

27. Inhalator gemäss einem der Ansprüche 21 bis 26, worin die inflammatorische Störung der Atemwege Asthma oder COPD ist.

## Revendications

1. Composition pharmaceutique comprenant un composé de formule (I) : 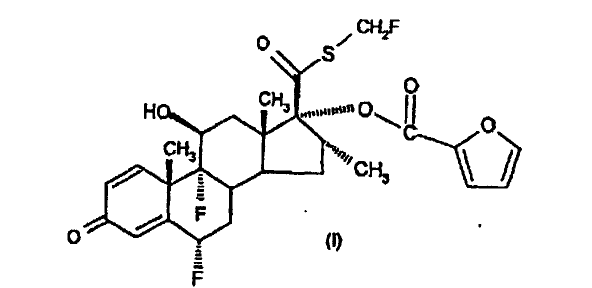 ou un solvate de celui-ci, en association avec un antagoniste de récepteur muscarinique.

2. Composition pharmaceutique selon la revendication 1, dans laquelle l'antagoniste de récepteur muscarinique est sélectif pour les récepteurs M1 et M3 de préférence au récepteur M2.

3. Composition pharmaceutique selon la revendication 1, dans laquelle l'antagoniste de récepteur muscarinique est l'ipratropium ou un sel de celui-ci, ou l'oxitropium ou un sel de celui-ci.

4. Composition pharmaceutique selon la revendication 1 ou la revendication 2, dans laquelle l'antagoniste de récepteur muscarinique a une durée d'action suffisante pour fournir un effet thérapeutique quand elle est administrée une seule fois ou deux fois par jour.

5. Composition pharmaceutique selon la revendication 4, dans laquelle l'antagoniste de récepteur muscarinique est le tiotropium ou un sel de celui-ci.

6. Composition pharmaceutique selon la revendication 1, dans laquelle l'antagoniste de récepteur muscarinique est un composé de formule (A) : 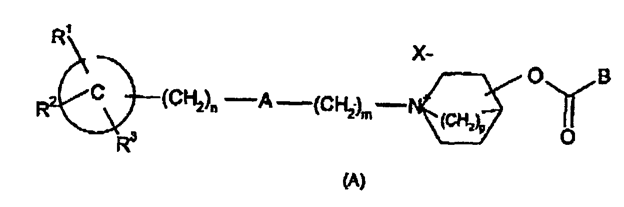 dans laquelle :
C est un cycle phényle, un groupe hétéroaromatique en C₄ à C₉ contenant un ou plusieurs hétéroatomes (choisis de préférence parmi les atomes d'azote, d'oxygène et de soufre), ou un groupe naphtalényle, 5,6,7,8-tétrahydronaphtalényle ou biphényle ;
R¹, R² et R³ représentent chacun indépendamment un atome d'hydrogène ou d'halogène, ou un groupe hydroxy ou un groupe phényle, -OR⁴, -SR⁴, -NR⁴R⁵, -NHCOR⁴, -CONR⁴R⁵, -CN, -NO₂, -COOR⁴ ou -CF₃, ou un groupe alkyle inférieur linéaire ou ramifié qui peut éventuellement être substitué, par exemple, par un groupe hydroxy ou alcoxy, où R⁴ et R⁵ représentent chacun indépendamment un atome d'hydrogène, un groupe alkyle inférieur linéaire ou ramifié, ou bien forment ensemble un noyau alicyclique ; ou bien R¹ et R² forment ensemble un cycle aromatique, alicyclique ou hétérocyclique ;
n est un nombre entier de 0 à 4 ;
A représente un groupe -CH₂-, -CH=CR⁶-, -CR⁶=CH-, -CR⁶R⁷-, -CO-, -O-, -S-, -S(O)-, SO₂ ou -NR⁶-, où R⁶ et R⁷ représentent chacun indépendamment un atome d'hydrogène, un groupe alkyle inférieur linéaire ou ramifié, ou bien R⁶ et R⁷ forment ensemble un noyau alicyclique ;
m est un nombre entier de 0 à 8 ; à la condition que lorsque m est égal à 0, A ne soit pas -CH₂- ;
p est un nombre entier de 1 à 2 et la substitution dans le cycle azoniabicyclique peut être aux positions 2, 3 ou 4 et comprendre toutes les configurations possibles des atomes de carbone asymétriques ;
B représente un groupe de formule (i) ou (ii) : 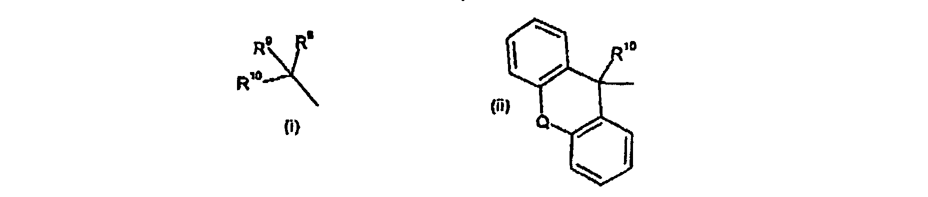 dans lesquelles R¹⁰ représente un atome d'hydrogène, un groupe hydroxy ou méthyle ; et R⁸ et R⁹ représentent chacun indépendamment l'une des 5 parties suivantes : 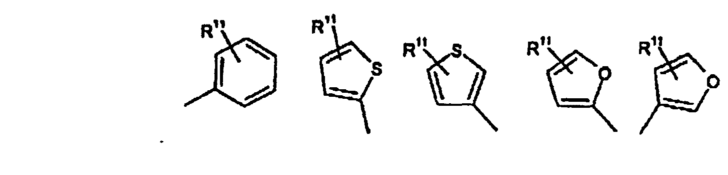 dans lesquelles R¹¹ représente un atome d'hydrogène ou d'halogène, ou un groupe alkyle inférieur linéaire ou ramifié et Q représente une simple liaison, -CH₂-, -CH₂-CH₂-, -O-, -O-CH₂-, -S-, -SCH₂- ou -CH=CH-, et quand (i) ou (ii) contiennent un centre chiral ils peuvent représenter l'une ou l'autre configuration ;
X représente un anion acceptable du point de vue pharmaceutique d'un acide monovalent ou polyvalent.

7. Composition pharmaceutique selon la revendication 6, dans laquelle C représente un groupe phényle, pyrrolyle ou thiènyle ; R¹, R² et R³ représentent chacun indépendamment un atome d'hydrogène, un groupe hydroxy ou un atome d'halogène ; n= 0 ou 1 ; m est un nombre entier de 1, 2 ou 3 ; A représente un groupe -CH₂-, -CH-CH- ou -O- ; p= 2 et le groupe substituant -OC(O)B attaché à l'azoniabicyclo[2.2.2]octane est à la position 3 de configuration (R); le groupe -OC(O)B est un groupe diphénylacétoxy, 2-hydroxy-2,2-diphényl-acétoxy, 2,2-diphénylpropionyloxy, 2-hydroxy-2-phényl-2-thièn-2-yl-acétoxy, 2-furan-2-yl-2-hydroxy-2-phénylacétoxy, 2,2-dithièn-2-ylacétoxy, 2-hydroxy-2,2-dithièn-2-ylacétoxy, 2-hydroxy-2,2-dithièn-3-ylacétoxy, 9-hydroxy-9[H]-fluorène-9-carbonyloxy, 9-méthyl-9[H]-fluorène-9-carbonyloxy, 9[H]-xanthène-9-carbonyloxy, 9-hydroxy-9[H]-xanthène-9-carbonyloxy ou 9 -méthyl-9[H]-xanthène-9-carbonyloxy ; et le groupe azoniabicyclo est substitué sur l'atome d'azote avec un groupe 3-phénoxypropyle, 2-phénoxypropyle, 3-phénylallyle, phénéthyle, 4-phénylbutyle, 3-phénylpropyle, 3-[2-hydroxyphénoxy]propyle, 3-[4-fluorophénoxy]propyle, 2-benzyloxyéthyle, 3-pyrrol-1-ylpropyle, 2-thièn-2-yléthyle ou 3-thièn-2-ylpropyle.

8. Composition pharmaceutique selon l'une quelconque des revendications 1 à 7, dans laquelle le composé de formule (I) ou un solvate de celui-ci et l'antagoniste de récepteur muscarinique sont tous les deux présents sous une forme particulaire.

9. Composition pharmaceutique selon la revendication 8, qui comprend de plus un support particulaire.

10. Composition pharmaceutique selon la revendication 9, dans laquelle le support est du lactose.

11. Composition pharmaceutique selon l'une quelconque des revendications 1 à 8, qui comprend de plus un gaz propulseur liquéfié.

12. Composition pharmaceutique selon l'une quelconque des revendications 1 à 11, dans laquelle le composé de formule (I) est sous une forme non solvatée.

13. Composition pharmaceutique selon la revendication 12, dans laquelle le composé de formule (I) est sous une forme non solvatée sous la forme polymorphe 1.

14. Composition pharmaceutique selon l'une quelconque des revendications 1 à 13, qui comprend de plus un agoniste d'adrénorécepteur β2 à longue durée d'action.

15. Composition pharmaceutique selon l'une quelconque des revendications 1 à 14, adaptée pour une administration par inhalation.

16. Composition pharmaceutique selon la revendication 15, utilisable dans le traitement des troubles inflammatoires et allergiques des voies respiratoires.

17. Utilisation d'une composition pharmaceutique selon l'une quelconque des revendications 1 à 14, pour la préparation d'un médicament destiné au traitement d'un trouble inflammatoire des voies respiratoires qui comprend une administration par inhalation.

18. Utilisation de la composition pharmaceutique selon la revendication 17, d ans laquelle le trouble inflammatoire des voies respiratoires est le syndrome respiratoire obstructif chronique ou l'asthme.

19. Formulation selon l'une quelconque des revendications 1 à 16, utilisable comme médicament chez l'homme ou en médecine vétérinaire dans le traitement de patients souffrant d'états inflammatoires et/ou allergiques pour un traitement à raison d'une seule fois par jour.

20. Utilisation d'une formulation selon l'une quelconque des revendications 1 à 16, pour la fabrication d'un médicament destiné au traitement d'un patient souffrant d'un état inflammatoire et/ou allergique.

21. Inhalateur contenant une pluralité de doses d'une formulation pharmaceutique qui comprend un composé de formule (I) 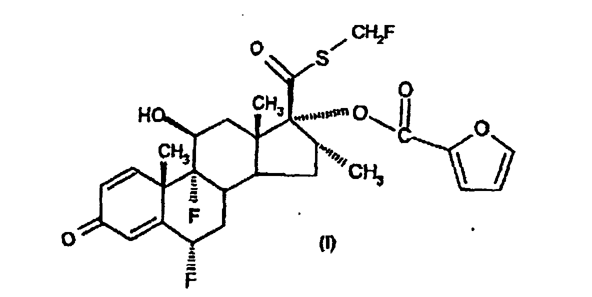 ou un solvate de celui-ci, en association avec un antagoniste de récepteur muscarinique à action de longue durée, laquelle formulation a un effet utile du point de vue thérapeutique dans le traitement des troubles inflammatoires des voies respiratoires pendant une période de 24 heures ou plus, et lesquelles doses conviennent pour une administration de la formulation par inhalation une seule fois par jour.

22. Inhalateur selon la revendication 21, dans lequel le composé de formule (I) ou un solvate de celui-ci et l'antagoniste des récepteurs muscariniques à action de longue durée sont tous les deux présents sous une forme particulaire.

23. Inhalateur selon la revendication 22, dans lequel la formulation comprend de plus un support particulaire.

24. Inhalateur selon la revendication 23, dans lequel le support est du lactose.

25. Inhalateur selon la revendication 21 ou 22, dans lequel la formulation comprend de plus un gaz propulseur liquéfié.

26. Inhalateur contenant une pluralité de doses d'une formulation pharmaceutique qui comprend un composé particulaire de formule (I) : 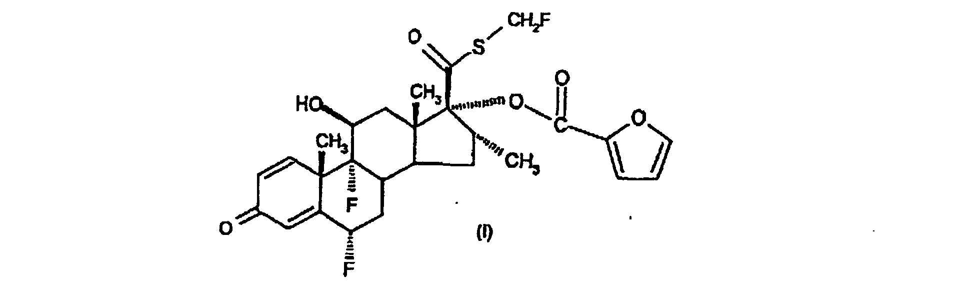 ou un solvate de celui-ci, un antagoniste particulaire de récepteur muscarinique à action de longue durée et un support, chaque médicament étant présent en une quantité adéquate pour fournir un effet utile du point de vue thérapeutique dans le traitement des troubles inflammatoires des voies respiratoires pendant une période de 24 heures ou plus à la suite d'une administration par inhalation une seule fois par jour.

27. Inhalateur selon l'une quelconque des revendications 21 à 26, où le trouble inflammatoire des voies respiratoires est l'asthme ou le syndrome respiratoire obstructif chronique (COPD).
